(19) Europäisches Patentamt
European Patent Office
Office européen des brevets

(11) Publication number: **0 575 845 A2**

# (12) EUROPEAN PATENT APPLICATION

(21) Application number: **93109447.8**

(51) Int. Cl.[5]: **C12Q 1/68**

(22) Date of filing: **14.06.93**

(30) Priority: **23.06.92 US 903028**

(43) Date of publication of application:
**29.12.93 Bulletin 93/52**

(84) Designated Contracting States:
**AT BE CH DE DK ES FR GB GR IE IT LI LU NL PT SE**

(71) Applicant: **F. HOFFMANN-LA ROCHE AG**
**Postfach 3255**
**CH-4002 Basel(CH)**

(72) Inventor: **Begovich, Ann B.**
**7306 Rockway Avenue**
**El Cerrito, California 94530(US)**
Inventor: **Bugawan, Teodorica L.**
**15524 Faris Street**
**San Leandro, California 94579(US)**
Inventor: **Erlich, Henry A.**
**3936 Rhoda Avenue**
**Oakland, California 94602(US)**

(74) Representative: **Wächter, Dieter Ernst, Dr. et al**
**P.O. Box 3255**
**CH-4002 Basel (CH)**

## (54) Method for HLA-DP typing.

(57) A process for determining an individual's HLA DP genotype from a nucleic acid containing sample obtained from the individual, which process comprises: (a) amplifying a target region of the nucleic acids in the sample under conditions suitable for carrying out a polymerase chain reaction, whereby the target region contains a polymorphic region (variable segment) of an HLA DP gene using a specific primer; (b) mixing the amplified nucleic acids with a panel of sequence specific oligonucleotide (SSO) probes, wherein each probe is complementary to a variant sequence of a variable segment of an HLA DP gene, under conditions wherein SSO probes bind to said amplified nucleic acids to form stable hybrid duplexes only if they are exactly complementary; and (c) detecting hybrids formed between the amplified nucleic acids and the SSO probes. The invention also relates to the said primers per se, to kits comprising said oligonucleotide primers and to oligonucleotide probes useful in HLA-DP DNA typing methods. These HLA-DP DNA typing methods may be useful in the prevention of graft rejection and graft versus host disease, in determining susceptibility to autoimmune diseases, in providing evidence concerning the derivation from an individual of forensic samples, and in paternity testing.

EP 0 575 845 A2

This invention relates to a method and compositions for determining the HLA-DP genotype of an individual using gene amplification methodology as disclosed and claimed in U.S. Patent Nos. 4,683,195 and 4,683,202 and the dot-blot and allele-specific oligonucleotide probe technology as disclosed and claimed in U.S. Patent No. 4,683,194. The methods and probes of the invention specifically relate to the detection of the polymorphic class II HLA-DP genes. The invention relates to the fields of molecular biology, diagnostic medicine, and forensics.

To aid in understanding the invention described below it is referred to the introductory part of the International Patent Application, Publication No. WO 89/11547, wherein an introduction in the fields mentioned above is given and wherein the definition of the terms used is the same as the definition of the terms used in the present specification.

WO 89/11547 discloses a process for determining an individual's HLA-DP genotype from a nucleic acid containing sample obtained from the individual comprising: (a) amplifying a target region of said nucleic acid which contains a polymorphic region of an HLA-DP gene; (b) hybridizing the amplified nucleic acid with a panel of sequence specific oligonucleotide (SSO) probes specific for variant segments of HLA-DP genes under conditions that allow each of said SSO probes to form a stable hybrid duplex with the amplified nucleic acid only if the complementary sequence is contained in the amplified nucleic acid; and (c) detecting hybrids formed between the amplified nucleic acid and the SSO probes.

WO 89/11547 describes also kits useful for determining the HLA-DP genotype of an individual; these kits comprise a panel of SSO probes for allelic variant sequences in said target region; and (b) instructions for determining the genotype by utilizing kit ingredients.

The present invention provides improved processes and reagents for determining the HLA-DP genotype of an individual from a nucleic acid containing sample obtained from said individual. The invention results from the discovery and characterization of further sequence polymorphisms in the variable second exon of the HLA-DPB1 alleles. As a result, further DPB1 (DPbeta) allelic variants have been discovered, viz. the alleles DPB21, DPB22, DPB23, DPB24, DPB25, DPB26, DPB27, DPB28, DPB29, and DPB30 described below. Based upon the novel sequence of these DP genes, the sequences of SSO probes are provided for the detection of the variant genotypes. The variations between the different DP alleles are dispersed. Therefore, one probe alone is rarely able to identify uniquely a specific DPB1 allele. Rather, the identity of an allele is inferred from the pattern of binding of a panel of probes, with each individual probe of the panel specific to different segments of a DPB1 gene.

More specifically the present invention relates to a process for determining an individual's HLA DP genotype from a nucleic acid containing sample originating from the individual whose HLA-DP genotype is to be determined, which method comprises amplifying a target region of the nucleic acids in the sample under conditions suitable for carrying out a polymerase chain reaction, whereby the target region contains a polymorphic region (variable segment) of an HLA DP gene using a primer selected from the group consisting of the primers

AB111 SEQ ID NO: 91 5'GGGATCCGAGAGTGGCGCCTCCGCTCAT,
RS348 SEQ ID NO: 142 5'ACACAGGAAACAGCTATGACCATG, and
RS349 SEQ ID NO: 143 5'CCAGGGTTTTCCCAGTCACGAC;

mixing the amplified nucleic acids with a panel of sequence specific oligonucleotide (SSO) probes, wherein each probe is complementary to a variant sequence of a variable segment of an HLA DP gene, under conditions wherein SSO probes bind to said amplified nucleic acids to form stable hybrid duplexes only if they are exactly complementary; and detecting hybrids formed between the amplified nucleic acids and the SSO probes. Said probes are preferably selected from the group consisting of:

SEQ ID NO: 92 (5'CCTGATGAGGTGTACTG);
SEQ ID NO: 99 (5'GAATTACCTTTTCCAGGGA);
SEQ ID NO: 100 (5'ATTACGTGTACCAGTTACG);

SEQ ID NO: 101 (5'CGTCCCTGGTACACGTAAT);
SEQ ID NO: 102 (5'CCTGCTGCGGAGTACTG);
SEQ ID NO: 103 (5'CAGTACTCCTCATCAGG);
SEQ ID NO: 104 (5'CAGTACTCCGCCTCAGG);
SEQ ID NO: 105 (5'CCTGATGAGGACTACTG);
SEQ ID NO: 106 (5'GACATCCTGGAGGAGAAGC);
SEQ ID NO: 107 (5'GCTCCTCCTCCAGGATGTC);
SEQ ID NO: 108 (5'GACCTCCTGGAGGAGAAGC);
SEQ ID NO: 109 (5'GCTCCTCCTCCAGGAGGTC);
SEQ ID NO: 110 (5'ATTACGTGCACCAGTTACG);
SEQ ID NO: 111 (5'CGTAACTGGTACACGTAAT);
SEQ ID NO: 112 (5'CTGCAGGGTCATGGGCCCCCG);
SEQ ID NO: 113 (5'CTGCAGGGTCACGGCCTCGTC);
SEQ ID NO: 114 (5'ATTACGTGTACCAGTTA);
SEQ ID NO: 115 (5'CCTGAGGCGGAGTACTG);
SEQ ID NO: 116 (5'GACCTCCTGGAGGAGGAG);
SEQ ID NO: 117 (5'GACCTCCTGGAGGAGAGG);
SEQ ID NO: 118 (5'CTGGTCGGGCCCATGACC);
SEQ ID NO: 119 (5'GAATTACCTTTTCCAGGGAC);
SEQ ID NO: 120 (5'TTACGTGTACCTGGGAC);
SEQ ID NO: 121 (5'ACATCCTGGAGGAGAAGC);
SEQ ID NO: 122 (5'ACATCCTGGAGGAGGAGC);
SEQ ID NO: 123 (5'ACCTCCTGGAGGAGAAGC);
SEQ ID NO: 124 (5'CCTGATGAGGAGTACTG);
SEQ ID NO: 125 (5'CTGGGCGGGCCCATG);
SEQ ID NO: 126 (5'CTGGACGAGGCCGTG);
SEQ ID NO: 127 (5'GACCTCCTGGAGGAGGAGC);
SEQ ID NO: 128 (5'GACCTCCTGGAGGAGAGGC);
SEQ ID NO: 129 (5'AGCTGGGCGGGCCCATGAC);
SEQ ID NO: 130 (5'AGCTGGACGAGGCCGTGAC);
SEQ ID NO: 132 (5'ATTACGTGCACCAGTTAC);
SEQ ID NO: 133 (5'ATTACGTGCACCAGTTA);
SEQ ID NO: 134 (5'CAGTACTCCTCATCAG);
SEQ ID NO: 135 (5'CTGATGAGGACTACTG);
SEQ ID NO: 137 (5'CCGTCCCTGGAAAAGGTAATTC);
SEQ ID NO: 138 (5'GACCTCCTGNGAGGAGAGGC);
SEQ ID NO: 139 (5'GACCTCCTGGAGNGAGGAGC);
SEQ ID NO: 151 (X-AGGAGTTCGCGCGCTT);

SEQ ID NO: 152 (X-AGGAGTTCGTGCGCTT);
SEQ ID NO: 153 (X-AGGAGCTCGTGCGCTTC);
SEQ ID NO: 154 (X-CCGGCAGGAGTACGCGC);
SEQ ID NO: 155 (X-GAGGAGTACGCGCGCT);
SEQ ID NO: 156 (X-CGAGCTGGGCGGGCCCA); and
SEQ ID NO: 157 (X-CGAGCTGGTCGGGCCCA),

more preferably they are selected from the group of novel probes having the nucleic acid sequences:

SEQ ID NO: 92 (5'CCTGATGAGGTGTACTG);
SEQ ID NO: 132 (5'ATTACGTGCACCAGTTAC);
SEQ ID NO: 133 (5'ATTACGTGCACCAGTTA);
SEQ ID NO: 134 (5'CAGTACTCCTCATCAG);
SEQ ID NO: 135 (5'CTGATGAGGACTACTG);
SEQ ID NO: 137 (5'CCGTCCCTGGAAAAGGTAATTC);
SEQ ID NO: 138 (5'GACCTCCTGNGAGGAGAGGC);
SEQ ID NO: 139 (5'GACCTCCTGGAGNGAGGAGC);
SEQ ID NO: 151 (X-AGGAGTTCGCGCGCTT);
SEQ ID NO: 152 (X-AGGAGTTCGTGCGCTT);
SEQ ID NO: 153 (X-AGGAGCTCGTGCGCTTC);
SEQ ID NO: 154 (X-CCGGCAGGAGTACGCGC);
SEQ ID NO: 155 (X-GAGGAGTACGCGCGCT);
SEQ ID NO: 156 (X-CGAGCTGGGCGGGCCCA); and
SEQ ID NO: 157 (X-CGAGCTGGTCGGGCCCA).

In the preferred process in accordance with the present invention the panel of probes comprises probes with hybridizing regions:

SEQ ID NO: 88 (5'TGTCTGCACATCCTGTCCG);
SEQ ID NO: 89 (5'TGTCTGCATACCCTGTCCG);
SEQ ID NO: 90 (5'CGGACAGGATATGCAGACA);
SEQ ID NO: 99 (5'GAATTACCTTTTCCAGGGA);
SEQ ID NO: 101 (5'CGTCCCTGGTACACGTAAT);
SEQ ID NO: 102 (5'CCTGCTGCGGAGTACTG);
SEQ ID NO: 105 (5'CCTGATGAGGACTACTG);
SEQ ID NO: 106 (5'GACATCCTGGAGGAGAAGC);
SEQ ID NO: 107 (5'GCTCCTCCTCCAGGATGTC);
SEQ ID NO: 108 (5'GACCTCCTGGAGGAGAAGC);
SEQ ID NO: 110 (5'ATTACGTGCACCAGTTACG);
SEQ ID NO: 112 (5'CTGCAGGGTCATGGGCCCCG);
SEQ ID NO: 114 (5'ATTACGTGTACCAGTTA);

SEQ ID NO: 115 (5'CCTGAGGCGGAGTACTG);
SEQ ID NO: 116 (5'GACCTCCTGGAGGAGGAG);
SEQ ID NO: 117 (5'GACCTCCTGGAGGAGAGG);
SEQ ID NO: 126 (5'CTGGACGAGGCCGTG); and
SEQ ID NO: 131 (5'CCAGTACTCCTCATCAGGC),

most prefereably said panel of probes further comprises a probe with hybridising region SEQ ID NO: 92 (5'CCTGATGAGGTGTACTG).

The present invention relates also to the novel primers

AB111 SEQ ID NO: 91 5'GGGATCCGAGAGTGGCGCCTCCGCTCAT,
RS348 SEQ ID NO: 142 5'ACACAGGAAACAGCTATGACCATG, and
RS349 SEQ ID NO: 143 5'CCAGGGTTTTCCCAGTCACGAC per se,

and to the novel probes mentioned above per se, especially when used as a diagnostic tool, e.g. for determining an individual's HLA DP genotype, such as in the case wherein the individual's HLA DP genotype comprises an allele selected from the group consisting of DPB21, DPB22, DPB23, DPB24, DPB25, DPB26, DPB27, DPB28, DPB29, and DPB30.

Because the present invention also is compatible with amplified nucleic acids, and because the PCR technique can amplify extremely small quantities of nucleic acid, samples containing vanishingly small amounts of nucleic acid can be typed for the presence of particular HLA-DP variants by the method of the present invention. For instance, even a single hair, contains enough DNA for purposes of the present invention, as evidenced by the work with DQalpha described by Higuchi et al., 1988, Nature 332:543-546.

In general, the nucleic acid in the sample will be DNA, most usually genomic DNA. However, the present invention can also be practiced with other nucleic acids, such as messenger RNA or cloned DNA, and the nucleic acid may be either single-stranded or double-stranded in the sample and still be suitable for purposes of the present invention. Those of skill in the art recognize that whatever the nature of the nucleic acid, the nucleic acid can be typed by the present method merely by taking appropriate steps at the relevant stage of the process. When PCR is used to amplify the nucleic acid in the sample, then the sample will usually comprise double-stranded DNA when typed with the novel probes of the invention.

As noted above, the HLA-DP typing method and probes of the invention are used in conjunction with PCR-amplified target DNA. Those practicing the present invention should note, however, that amplification of HLA-DP target sequences in a sample may be accomplished by any known method which provides sufficient amplification so that the target sequence may be detected by nucleic acid hybridization to an SSO probe. Although the PCR process is well known in the art (see U.S. Patent Nos. 4,683,195 and 4,683,202) and although a variety of commercial vendors, such as Perkin Elmer (Norwalk, CT), sell PCR reagents and publish PCR protocols, some general PCR information is provided below for purposes of clarity and full understanding of the invention to those unfamiliar with the PCR process.

To amplify a target nucleic acid sequence in a sample by PCR, the sequence must be accessible to the components of the amplification system. In general, this accessibility is ensured by isolating the nucleic acids from the sample. A variety of techniques for extracting nucleic acids from biological samples are known in the art. For example, see those described in Maniatis et al., Molecular Cloning: A Laboratory Manual, (New York, Cold Spring Harbor Laboratory, 1982). Alternatively, if the sample is fairly readily disruptable, the nucleic acid need not be purified prior to amplification by the PCR technique, i.e., if the sample is comprised of cells, particularly peripheral blood lymphocytes or amniocytes, lysis and dispersion of the intracellular components may be accomplished merely by suspending the cells in hypotonic buffer.

Because the nucleic acid in the sample is first denatured (assuming the sample nucleic acid is double-stranded) to begin the PCR process, and because simply heating some samples results in the disruption of cells, isolation of nucleic acid from the sample can sometimes be accomplished in conjunction with strand separation. Strand separation can be accomplished by any suitable denaturing method including physical, chemical, or enzymatic means. Typical heat denaturation involves temperatures ranging from about 80°C to 105°C for times ranging from about 1 to 10 minutes. Strand separation may also be induced by a helicase, an enzyme capable of exhibiting helicase activity. For example, the enzyme RecA has helicase activity in the presence of ATP. The reaction conditions suitable for strand separation by helicases are known in the art (see Kuhn Hoffman-Berling, 1978, CSH-Quantitative Biology 43:63, and Radding, 1982, Ann. Rev. Genetics 16:405-436).

As noted above strand separation may be accomplished in conjunction with the isolation of the sample nucleic acid or as a separate step. In this embodiment of the PCR process, the reaction is catalyzed by a heat-stable polymerase and carried out at an elevated temperature. The temperature is one at which the enzyme is thermostable, and at which the nucleic acids are in an equilibrium of single and double strands, so that sufficient primer will anneal to template strands to allow a reasonable rate of polymerization. In the preferred embodiment of the PCR process, however, strand separation is achieved by heating the reaction to a sufficiently high temperature for an effective time to cause the denaturation of the duplex, but not to cause an irreversible denaturation of the polymerase (see European Patent Application, Publication No. 258,017, incorporated herein by reference).

Once the strands are separated, the next step in PCR involves hybridizing the separated strands with primers that flank the target sequence. The primers are then extended to form complementary copies of the target strands, and the cycle of denaturation, hybridization, and extension is repeated as many times as necessary to obtain the desired amount of amplified nucleic acid.

As noted above, the present invention also provides PCR novel primers for HLA-DP DNA amplification and typing. These primers are complementary to sequences in the conserved regions that flank the target sequences in the variant regions of the HLA-DP loci. For purposes of the present invention, the preferred variant region of the HLA-DP loci is the second exon of the DPA1 and DPB1 genes. For successful PCR amplification, the present primers are designed so that the position at which each primer hybridizes along a duplex sequence is such that an extension product synthesized from one primer, when it is separated from its template (complement), serves as a template for the extension of the other primer to yield an amplified segment of nucleic acid of defined length. Moreover, primers are provided that will bind preferentially to the HLA-DP region under selective annealing conditions. Preferred primers are

UG19 SEQ ID NO: 144 (GCTGCAGGAGAGTGGCGCCTCCGCTCAT) and
UG21 SEQ ID NO: 145 (CGGATCCGGCCCAAAGCCCTCACTC).

Template-dependent extension of primers in PCR is catalyzed by a polymerizing agent in the presence of adequate amounts of four deoxyribonucleoside triphosphates (dATP, dGTP, dCTP, and dTTP or dUTP) in a reaction medium comprised of the appropriate salts, metal cations, and pH buffering system. Suitable polymerizing agents are enzymes known to catalyze template-dependent DNA synthesis. For example, if the template is RNA, a suitable polymerizing agent to convert the RNA into a complementary DNA (cDNA) sequence is reverse transcriptase (RT), such as avian myeloblastosis virus RT. Once the target for amplification is DNA, suitable polymerases include, for example, E. coli DNA polymerase I or its Klenow fragment, $T_4$ DNA polymerase, and Taq polymerase, a heat stable DNA polymerase isolated from Thermus aquaticus and commercially available from Perkin-Elmer (Norwalk, CT). The latter enzyme is widely used in the amplification and sequencing of nucleic acids. The reaction conditions for using DNA polymerases are known in the art, and are described in, for example, the treatise Methods in Enzymology, and in Maniatis et al., Molecular Cloning: A Laboratory Manual, supra.

The PCR method can be performed in a step-wise fashion, where after each step new reagents are added, or in a fashion where all of the reagents are added simultaneously, or in a partial step-wise fashion, where fresh or different reagents are added after a given number of steps. For example, if strand separation is induced by heat, and the polymerase is heat-sensitive, then the polymerase will have to be added alter every round of strand separation. However, if, for example, a helicase is used for denaturation, or if a thermostable polymerase is used for extension, then all of the reagents may be added initially, or, alternatively, if molar ratios of reagents are of consequence to the reaction, the reagents may be replenished periodically as they are depleted by the synthetic reaction.

Due to the enormous amplification possible with the PCR process, small amounts of DNA carried over from other samples, positive control templates, or from previous amplifications can provide enough template to result in PCR product, even in the absence of added template DNA. If possible, all reaction mixes are set up in an area separate from PCR product analysis and sample preparation. The use of dedicated or disposable vessels, solutions, and pipettes (preferably positive displacement pipettes or plugged pipette tips) for RNA/DNA preparation, reaction mixing, and sample analysis will minimize cross contamination. See also Higuchi and Kwok, 1989, Nature, 339: 237-238, and Kwok, and Orrego, in Innis et al. eds., 1990 PCR Protocols: A Guide to Methods and Applications, Academic Press, Inc., San Diego, CA, which are incorporated herein by reference.

One particular method for minimizing the effects of cross contamination of nucleic acid amplification is described in International Patent Application, Publication No. WO 92/01814, which is incorporated herein by reference. The method involves the introduction of unconventional nucleotide bases, such as dUTP, into the amplified product. Exposure of the amplification product to enzymatic and/or physical-chemical treatment renders the product DNA incapable of serving as a template for subsequent amplifications. For example, uracil-DNA glycosylase will remove uracil residues from PCR product containing that base. Enzyme treatment of a PCR reaction mixture prior to amplification results in the degradation of any contaminating uracil-containing PCR product from a prior reaction and serves to "sterilize" the amplification reaction.

It is preferable, but not essential, to add the thermostable DNA polymerase to the reaction mix after both the primer and the template are added. By separating at least one component that is essential for primer extension, the initiation of polymerization can be controlled and non-specific primer hybridization and extension is minimized. Polymerization initiation can also be controlled by delaying the addition of $MgCl_2$. A modification of PCR referred to as "hot start" is described in International Patent Application, Publication No. WO 91/12342, which is incorporated herein by reference. In hot start PCR, the addition of polymerase is delayed until the initial high-temperature denaturation step, thereby minimizing the formation of extension products from non-specific primer hybridization which may occur if all reaction components are added at room temperature.

Those skilled in the art will know that the PCR process is usually carried out as an automated process with a thermostable enzyme. In this process, the reaction mixture is cycled through a denaturing region, a primer annealing region, and a reaction region. A machine specifically adapted for use with a thermostable enzyme is disclosed more completely in European Patent Application, Publication No. 236,069, incorporated herein by reference, and is commercially available from Perkin Elmer (Norwalk, CT).

One reason, as noted above, the PCR process is important in the method of the present invention is that the PCR process can be used to amplify the sample nucleic acid prior to HLA-DP DNA typing. Another important use of PCR for purposes of the present invention, however is for determining the nucleotide sequence of previously undiscovered allelic variants which exist in the HLA-DP region, so that probes for those variants can be constructed and used in the present method. In this use of the PCR process, polymorphic regions of the DPA1 and DPB1 genes are amplified, and the nucleotide sequences of these polymorphic target regions, for example, the second exon of the DPA1 and DPB1 genes, are determined. As illustrated below, it is also useful for the cells containing a particular variant to be typed by serological typing, mixed lymphocyte typing, or primed lymphocyte typing to correlate the nucleotide sequence of a particular variant with the DP type established by prior art methods.

Analysis of the nucleotide sequence of the target region of a DP variant allele can be readily performed by direct analysis of the PCR products. A preferred sequencing protocol is described in Innis et al., 1988, Proc. Natl. Acad. Sci. 85:9436-9440, and U.S. Patent No. 5,075,216, incorporated herein by reference. A process for direct sequence analysis of PCR amplified products is also described by Saiki et al., 1988, Science 239:487-491. Alternatively, the amplified target sequence may be cloned prior to sequence analysis, as described by Scharf et al., 1986, Science 233:1076-1078.

As discussed in WO 89/11547, a panel of DPw typed cells, representing a large number of different haplotypes, has been analyzed by PCR and nucleotide sequencing of the second exon of the DPB1 gene. As a result of this effort and similar efforts using samples obtained from a variety of individuals suffering from autoimmune diseases, a large number of different allelic variants in this locus were discovered. In general, the results demonstrated that specific DPB1 sequences correlate with the standard PLT-defined DPw1 through DPw6 specificities. The rare exceptions may reflect the difficulty of obtaining a standard and reproducible PLT DPw typing system, which difficulties highlight the advantages of the present method. In this regard, it is relevant that the cell line Cox, originally typed as DPw1, has been retyped as DPw3 and contains the DPB3 allele (now DPB1*0301, the new nomenclature is listed elsewhere).

Thus, the present invention also relates to a process for determining an individual's susceptibility to an autoimmune disease comprising determining the individual's HLA DP genotype in accordance with the present invention and determining whether the individual's genotype is a genotype which is linked to an autoimmune disease. Said autoimmune disease is either linked to the DPB2.1 allele, such as in the case of pauciarticular juvenile rheumatoid arthritis and insulin dependent diabetes mellitus (IDDM), or to an allele selected from the group consisting of DPB13, DPB1, DPB3, and DPB4.2, such as in the case of coeliac disease (CD).

For other interesting and important correlations between the serologically defined DP types and DP variant nucleotide sequences and for descriptions of how to perform HLA-DNA typing on the DPA1 (previously DPalpha) locus and on the DPB1 (previously DPbeta) locus see WO 89/11547.

The DNA sequences of the DPA1 and DPB1 genes serve as a useful starting point in the design of the sequence specific oligonucleotide probes of the present invention. These probes are designed so that, under stringent hybridization conditions, the probes hybridize specifically only to exactly complementary sequences in variant segments of the DPA1 and DPB1 alleles. These SSO probes may be of any length which spans the variant sequences in a variant region and allows for sequence specific hybridization, but preferably the hybridizing region of the probe is short, in the range of 10 to 30 bases, and more preferably is about 17 to 19 bases in length. For immobilization, the probe may also contain long stretches of poly-T which can be fixed to a solid support by irradiation, a technique described in more detail in International Patent Application, Publication No. WO 89/11548 and in European Patent Application, Publication No. 237,362. The disclosures of these applications are incorporation herein by reference.

The SSO probes of the invention are also designed to hybridize specifically with a particular variant segment of a DP allele and to have destabilizing mismatches with the other variant sequences known for the particular segment. Preferably, the probes are specific for variant DNA segments in the variable second exons of the DPA1 and DPB1 genes, and even more preferably, the probes are specific for DNA segments encoding the residues near positions 8-11, 36, 55-57, 65-69, 76, and 84-87 of the second exon. Oligonucleotide probes which have been designed to hybridize specifically to the second exons of the DPB1 and DPA1 alleles are described in more detail below and in the Examples.

The probes of the invention can be synthesized and labeled using the techniques described above in the discussion of PCR primers. For example, the probe may be labeled at the 5'-end with $^{32}P$ by incubating the probe with $^{32}P$-ATP and kinase. A suitable non-radioactive label for SSO probes is horseradish peroxidase (HRP). Methods for preparing and detecting probes containing this label are described in the Examples below and in European Patent Application, Publication No. 237,362, and incorporated herein by reference. For additional information on the use of such labeled probes, see U.S. Patent No. 4,789,630; Saiki et al., 1988, N. Eng. J. Med. 319:537-541; and Bugawan et al., 1988, Bio/Technology 6:943-947, incorporated herein by reference. Useful chromogens include red leuco dye and tetramethyl benzidine (TMB).

The probes of the invention can be used to identify the allelic sequences present in a sample by determining which of the SSO probes bind to the HLA-DP sequences present in the sample. Suitable assay methods for purposes of the present invention to detect hybrids formed between SSO probes and nucleic acid sequences in a sample are known in the art. For example, the detection can be accomplished using a dot blot format, as described in the Examples. In the dot blot format, the unlabeled amplified sample is bound to a membrane, the membrane incubated with labeled probe under suitable hybridization conditions, unhybridized probe removed by washing, and the filter monitored for the presence of bound probe. When multiple samples are analyzed with few probes, a preferred process requires high stringency hybridization and wash conditions, allowing only perfectly matched hybrids to exist.

An alternative method is a "reverse" dot blot format, in which the amplified sequence contains a label. In this format, the unlabeled SSO probes are bound to the membrane and exposed to the labeled sample under appropriately stringent hybridization conditions. Unhybridized labeled sample is then removed by washing under suitably stringent conditions, and the filter is then monitored for the presence of bound sequences.

In another version of the "reverse" dot blot format, the SSO probe is labeled, and the sample nucleic acid is unlabeled. After hybridization and washing, the labeled probe, or a labeled fragment of the probe, is released from the membrane and detected to determine if a sequence in the sample hybridized to the labeled oligonucleotide. Release of label can be achieved by digestion with a restriction enzyme that recognizes a restriction site in the duplex hybrid. This procedure, known as oligomer restriction, is described more fully in U.S. Patent No. 4,683,194 and corresponding EP Patent Publication 164,054, each of which are incorporated herein by reference.

Whatever the method for determining which DP SSO probes of the invention hybridize to DP sequences in a sample, the central feature of the DP-DNA typing method involves the identification of the HLA-DP alleles present in the sample by analyzing the pattern of binding of a panel of SSO probes. Although single probes of the invention can certainly be used to provide useful information, the variation in the DPB1 alleles is dispersed in nature, so rarely is any one probe able to identify uniquely a specific DP variant. Rather, as shown in the Examples, the identity of an allele is inferred from the pattern of binding of a panel of SSO probes, which are specific to different segments of the DPA1 and DPB1 genes.

DNA typing of HLA-DP alleles is useful for many different purposes, e.g. for determining an individual's susceptibility to autoimmune diseases linked to certain HLA-DP alleles, as discussed in detail in WO 89/11547 and below. It is anticipated that as medical technology develops, more disease or disease-prone states, including Grave's disease, S.L.E., and Shögren's Syndrome, will become known to be associated with various DP alleles. The present invention provides methods for distinguishing such alleles from other alleles and so provides a means to identify individuals at high risk for an autoimmune disease. In a preferred embodiment, an individual whose susceptibility is to be determined is analyzed for HLA-DP type first by using the PCR method to amplify the target region of the HLA-DP locus. Then, SSO probes are hybridized to the amplified target region, and the particular DP allele present in the amplified DNA is determined from the pattern of binding of the SSO probes. Finally, one determines whether the allele present in the amplified DNA is an allele associated with the autoimmune disease.

The present method, however, is not limited to the field of medical science in ability to provide significant benefits. DNA typing methods also now play a significant role in the important area of individual identification, whether for solving crimes, as when the identity of a criminal or victim is established by linking an individual with evidence left at the scene of a crime, or for solving other issues of a non-criminal nature, as when biological material is used to determine the maternity or paternity of an individual. Thus, the present invention also relates to a process of providing forensic evidence concerning the derivation of a sample which contains genomic nucleic acids comprising determining the HLA DP genotypes of the sample and of a suspected individual, comparing the HLA DP genotypes of the individual and of the sample, and deducing whether the sample could have been derived from the individual.

Whatever the purpose for which the present invention is employed, the differences between various DP alleles is key to the success of the method. Amino acid sequences from each of the DP alleles (and the SXA and SXB pseudoalleles) is provided in the Sequence Listing section; nucleotide sequences are provided for the DPB1 alleles. Shown below are the sequence identification numbers for the nucleic acid and amino acid sequences for each of the alleles. Two equivalent designations are given for each of the DPB1 alleles; the second is the official name assigned by the WHO Nomenclature Committee.

| Allele | (WHO) | Amino Acid Sequence |
|---|---|---|
| DPA1 | DPA1*0101 | SEQ ID NO: 1 |
| DPA2 | DPA1*0201 | SEQ ID NO: 2 |
| SXA | | SEQ ID NO: 3 |
| SXB | | SEQ ID NO: 4 |

| | Allele | Nucleotide Sequence | Amino Acid Sequence |
|---|---|---|---|
| DPB1 | DPB1*0101 | SEQ ID NO: 5 | SEQ ID NO: 6 |
| DPB2.1 | DPB1*0201 | SEQ ID NO: 7 | SEQ ID NO: 8 |
| DPB2.2 | DPB1*0202 | SEQ ID NO: 9 | SEQ ID NO: 10 |
| DPB3 | DPB1*0301 | SEQ ID NO: 11 | SEQ ID NO: 12 |
| DPB4.1 | DPB1*0401 | SEQ ID NO: 13 | SEQ ID NO: 14 |
| DPB4.2 | DPB1*0402 | SEQ ID NO: 15 | SEQ ID NO: 16 |
| DPB5 | DPB1*0501 | SEQ ID NO: 17 | SEQ ID NO: 18 |
| DPB6 | DPB1*0601 | SEQ ID NO: 19 | SEQ ID NO: 20 |
| DBP8 | DPB1*0801 | SEQ ID NO: 21 | SEQ ID NO: 22 |
| DPB9 | DPB1*0901 | SEQ ID NO: 23 | SEQ ID NO: 24 |
| DPB10 | DPB1*1001 | SEQ ID NO: 25 | SEQ ID NO: 26 |
| DPB11 | DPB1*1101 | SEQ ID NO: 27 | SEQ ID NO: 28 |
| DPB13 | DPB1*1301 | SEQ ID NO: 29 | SEQ ID NO: 30 |
| DPB14 | DPB1*1401 | SEQ ID NO: 31 | SEQ ID NO: 32 |
| DPB15 | DPB1*1501 | SEQ ID NO: 33 | SEQ ID NO: 34 |
| DPB16 | DPB1*1601 | SEQ ID NO: 35 | SEQ ID NO: 36 |
| DPB17 | DPB1*1701 | SEQ ID NO: 37 | SEQ ID NO: 38 |
| DPB18 | DPB1*1801 | SEQ ID NO: 39 | SEQ ID NO: 40 |
| DPB19 | DPB1*1901 | SEQ ID NO: 41 | SEQ ID NO: 42 |
| DPB20 | DPB1*2001 | | SEQ ID NO: 43 |
| DPB21 | DPB1*2801 | SEQ ID NO: 44 | SEQ ID NO: 45 |
| DPB22 | DPB1*3101 | SEQ ID NO: 46 | SEQ ID NO: 47 |
| DPB23 | DPB1*2701 | SEQ ID NO: 48 | SEQ ID NO: 49 |
| DPB24 | DPB1*3201 | SEQ ID NO: 50 | SEQ ID NO: 51 |
| DPB25 | DPB1*3301 | SEQ ID NO: 52 | SEQ ID NO: 53 |
| DPB26 | DPB1*3401 | SEQ ID NO: 54 | SEQ ID NO: 55 |
| DPB27 | DPB1*2901 | SEQ ID NO: 56 | SEQ ID NO: 57 |
| DPB28 | DPB1*3001 | SEQ ID NO: 58 | SEQ ID NO: 59 |
| DPB29 | DPB1*3501 | SEQ ID NO: 60 | SEQ ID NO: 61 |
| DPB30 | DPB1*2101 | SEQ ID NO: 62 | SEQ ID NO: 63 |

The sequence information provided above is repeated in the amino acid and nucleotide sequence alignment Tables below in a form which permits easy visual inspection.

The most significant differences between DP alleles can be detected quite readily when the various amino acid sequences encoded by the alleles are aligned and examined. Such an alignment is shown below, where a dash indicates identity with the DPB4.1 allele (for the various DPB1 alleles and the DPB1 pseudogene, designated SXB) or with the DPA1 allele (for the DPA1 allele, DPA2, and the DPalpha pseudogene, designated SXA). In this depiction, the numbered positions are for the mature peptide subunits, allele designations are at left, and representative cell sources are at right.

```
DPalpha Alleles
              10        20        30        40        50        60        70        80
DPA1:   DHVSTYAAFVQTHRPTGEFMFEFDEDEMFYVDLDKKETVWHLEEFGQAFSFEAQGGLANIAILNNNLNTLIQRSNHTQATN   (LB)
DPA2:   ---------------------------Q------------------R--------------------------------A-   (Daudi)
(SXA):  -------E--------S--Y------E-Q---N--E--M--P-P--IHT-D-G--R-I-G-VMARKH---R-NG KQ-W--D

DPbeta Alleles
            10        20        30        40        50        60        70        80        90
DPB4.1   NYLFQGRQECYAFNGTQRFLERYIYNREEFARFDSDVGEFRAVTELGRPAAEYWNSQKDILEEKRAVPDRMCRHNYELGGPMTLQRR (HHK)
DPB4.2     ----------------------------V------------------DE----------------------------------   (APD)
DPB2.1   ------------------------------V------------------DE------------E-----------------------  (LB)
DPB2.2     ---------------------------LV------------------E-------------E---------------------   (QBL)
DPB8       ----------------------------V------------------DE------------E------V-------DEAV---   (Plaz)
DPB5       ---------------------------LV------------------E----------------------------DEAV---   (HAS)
DPB3       VY-L------------------------V------------------DED-------L----------V-------DEAV---   (SLE)
DPB6       VY-L------------------------V------------------DED-------L---E--------------DEAV---   (JMOS)
DPB11      VY-L---------------------Q-Y-----------------------------L---R--------------DEAV---   (CRK)
DPB13      VY-L-----------------------Y---------------------------------E------I-------DEAV---   (BH)
DPB1       VY-------------------------Y----------------------------------------V-------DEAV---   (LUY)
DPB10      VH-L------------------------V------------------DE------------E------V-------DEAV---   (BM21)
DPB9       VH-L------------------------V------------------DED-----------E------V-------DEAV---   (TOK)
DPB14      VH-L------------------------V------------------DED-------L----------V-------DEAV---   (8268)
DPB15      VY-----------------------Q-Y-----------------------------L---R--------------V------   (PLH)
DPB16      ----------------------------V------------------DE------------E--------------DEAV---   (JRA)
DPB17      VH-L------------------------V------------------DED-----------E--------------DEAV---   (JRAB)
DPB18      VY--------------------------V------------------DE---------------------------V------   (JCA)
DPB19      ----------------------------V------------------E-------------E------I-------DEAV---   (CB68)
DPB20      VH-L------------------------V------------------DED-------L------------------DEAV---
DPB21    -------------------------------------------------DE--------L------------------V------
DPB22    -----------------------------------------------------------L------L-----------DEAV---
DPB23    --VY-L-----------------------Y------------------------------------------------DEAV---
DPB24    ------------------------------V------------------DEV-----------E---------------------
DPB25    ---------------------------------------------------------------E---------------------
DPB26    -----------------------------LV----------------------------L------L-----------V------
DPB27    --VY-L------------------------V------------------DED-------L---E------V-------DEAV---
DPB28    --VH-L------------------------V------------------E-------------E--------------DEAV---
DPB29    --VH-L------------------------V------------------DED------------------V-------DEAV---
DBP30    --VY-L-----------------------LV------------------E-------------E--------------DEAV---
DPB2*    -SVY-E------------VVDGL------YVH--A----L--M------IG--F-----FM-R---EV-KV---K---ME-LIR---  (Priess)
```

To detect and distinguish between DP alleles in a practicable and economic fashion, however, one must know the nucleotide sequence of the alleles. Portions of the nucleotide sequences of various DPA1 and DPB1 alleles are shown below; the sequences are identified as above. The illustrative primers of the invention enable production of DNA from which the sequence of codons 8 to 90 can be determined. The location of allele sequences that are the preferred target sequences for hybridization with the various probes of the invention are designated as |-A-|, |-B-|, |-C-|, |-D-|, |-E-|, and |-F-|.

```
                          +10            +15            +20            +25
           AsnTyrLeuPheGlnGlyArgGlnGluCysTyrAlaPheAsnGlyThrGlnArgPheLeuGluArgTyr
DPB4.1:  AGAATTACCTTTTCCAGGGACGGCAGGAATGCTACGCGTTTAATGGGACACAGCGCTTCCTGGAGAGATAC
DPB4.2:           --------------------------------------------------------------
DPB2.1:  -----------------------------------------------------------------------
DPB2.2:           --------------------------------------------------------------
DPB8:             --------------------------------------------------------------
DPB7:             --------------------------------------------------------------
DPB3:             G-G-A----TT---------------------------------------------------
DPB6:             G-G-A----TT---------------------------------------------------
DPB11:            G-G-A----TT---------------------------------------------------
DPB13:            G-G-A----TT---------------------------------------------------
DPB1:             G-G-A---------------------------------------------------------
DPB9:             G-GCA----TT---------------------------------------------------
DPB10:            G-GCA----TT---------------------------------------------------
DPB14:            G-GCA----TT---------------------------------------------------
DPB15:            G-G-A---------------------------------------------------------
DPB16:            --------------------------------------------------------------
DPB17:            G-GCA----TT---------------------------------------------------
DPB18:            G-G-A---------------------------------------------------------
DPB19:            --------------------------------------------------------------
DPB20:            G-GCA----TT---------------------------------------------------
DPB21:   -----------------------------------------------------------------------
DPB22:   -----------------------------------------------------------------------
DPB23:   ---------G-G-A----TT---------------------------------------------------
DPB24:   -----------------------------------------------------------------------
DPB25:   -----------------------------------------------------------------------
DPB26:   -----------------------------------------------------------------------
DPB27:   ---------G-G-A----TT---------------------------------------------------
DPB28:   ---------G-GCA----TT------------------------------------------------
DPB29:   ---------G-GCA----TT---------------------------------------------------
DPB30:   ---------G-G-A----TT---------------------------------------------------
                |-----A-----|
```

```
           +30            +35             +40            +45            +50
         IleTyrAsnArgGluGluPheAlaArgPheAspSerAspValGlyGluPheArgAlaValThrGluLeu
DPB4.1:  ATCTACAACCGGGAGGAGTTCGCGCGCTTCGACAGCGACGTGGGGGAGTTCCGGGCGGTGACGGAGCTG
DPB4.2:  ----------------------T----------------------------------------------
DPB2.1:  ----------------------T----------------------------------------------
DPB2.2:  ------------------C---T----------------------------------------------
  DPB8:  ----------------------T----------------------------------------------
  DPB5:  ------------------C---T----------------------------------------------
  DPB3:  ----------------------T----------------------------------------------
  DPB6:  ----------------------T----------------------------------------------
 DPB11:  ------------C------A------------------------A------------------------
 DPB13:  -------------------A------------------------A------------------------
  DPB1:  -------------------A-------------------------------------------------
  DPB9:  ----------------------T----------------------------------------------
 DPB10:  ----------------------T----------------------------------------------
 DPB14:  ----------------------T----------------------------------------------
 DPB15:  ------------C------A------------------------A------------------------
 DPB16:  ----------------------T----------------------------------------------
 DPB17:  ----------------------T----------------------------------------------
 DPB18:  ----------------------T----------------------------------------------
 DPB19:  ----------------------T----------------------------------------------
 DPB20:  ----------------------T----------------------------------------------
 DPB21:  ---------------------------------------------------------------------
 DPB22:  ---------------------------------------------------------------------
 DPB23:  -------------------A-------------------------------------------------
 DPB24:  ----------------------T----------------------------------------------
 DPB25:  ---------------------------------------------------------------------
 DPB26:  ------------------C---T----------------------------------------------
 DPB27:  ----------------------T----------------------------------------------
 DPB28:  ----------------------T----------------------------------------------
 DPB29:  ----------------------T----------------------------------------------
 DPB30:  ------------------C---T----------------------------------------------

                    |-----B-----|
```

```
                   +55        +60        +65        +70
          GlyArgProAlaAlaGluTyrTrpAsnSerGlnLysAspIleLeuGluGluLysArgAlaValPro
DPB4.1:   GGGCGGCCTGCTGCGGAGTACTGGAACAGCCAGAAGGACATCCTGGAGGAGAAGCGGGCAGTGCCG
DPB4.2:   ----------A--A----------------------------------------------------
DPB2.1:   ----------A--A-------------------------------------G--------------
DPB2.2:   ----------AG---------------------------------------G--------------
  DPB8:   ----------A--A-------------------------------------G--------------
  DPB5:   ----------AG------------------------------------------------------
  DPB3:   ----------A--A---C---------------------C--------------------------
  DPB6:   ----------A--A---C---------------------C-----------G--------------
 DPB11:   ---------------------------------------C------------G-------------
 DPB13:   ---------------------------------------------------G--------------
  DPB1:   ------------------------------------------------------------------
  DPB9:   ----------A--A---C---------------------------------G--------------
 DPB10:   ----------A--A-------------------------------------G--------------
 DPB14:   ----------A--A---C---------------------C--------------------------
 DPB15:   ---------------------------------------C------------G-------------
 DPB16:   ----------A--A-------------------------------------G--------------
 DPB17:   ----------A--A---C---------------------------------G--------------
 DPB18:   ----------A--A----------------------------------------------------
 DPB19:   ----------AG---------------------------------------G--------------
 DPB20:   ----------A--A---C---------------------C--------------------------
 DPB21:   ----------A--A-------------------------C--------------------------
 DPB22:   ---------------------------------------C--------------------T-----
 DPB23:   ------------------------------------------------------------------
 DPB24:   ----------A--A---T---------------------------------G--------------
 DPB25:   ---------------------------------------------------G--------------
 DPB26:   ---------------------------------------C--------------------T-----
 DPB27:   ----------A--A---C---------------------C-----------G--------------
 DPB28:   ----------AG---------------------------------------G--------------
 DPB29:   ----------A--A---C------------------------------------------------
 DPB30:   ----------AG---------------------------------------G--------------

                   |---C----|                  |------D-------|
```

```
             +75           +80           +85            +90
          AspArgMetCysArgHisAsnTyrGluLeuGlyGlyProMetThrLeuGlnArgArg
DPB4.1:  GACAGGATGTGCAGACACAACTACGAGCTGGGCGGGCCCATGACCCTGCAGCGCCGAG
DPB4.2:  ----------------------------------------------------------
DPB2.1:  -------------------------------------------------
 PB2.2:  -------------------------------------------------
  DPB8:  ------G-A---------------------A--A-G--G----------
  DPB5:  ------------------------------A--A-G--G----------
  DPB3:  ------G-A---------------------A--A-G--G----------
  DPB6:  ------------------------------A--A-G--G----------
 DPB11:  ------------------------------A--A-G--G----------
 DPB13:  --------A---------------------A--A-G--G----------
  DPB1:  ------G-A---------------------A--A-G--G----------
  DPB9:  ------G-A---------------------A--A-G--G----------
 DPB10:  ------G-A---------------------A--A-G--G----------
 DPB14:  ------G-A---------------------A--A-G--G----------
 DPB15:  ------------------------------T------------------
 DPB16:  ------------------------------A--A-G--G----------
 DPB17:  ------------------------------A--A-G--G----------
 DPB18:  ------------------------------T------------------
 DPB19:  --------A---------------------A--A-G--G----------
 DPB20:  ------------------------------A--A-G--G----------
 DPB21:  ------------------------------T---------------------------
 DPB22:  ------------------------------A--A-G--G-------------------
 DPB23:  ------------------------------A--A-G--G-------------------
 DPB24:  ----------------------------------------------------------
 DPB25:  ----------------------------------------------------------
 DPB26:  ------------------------------T---------------------------
 DPB27:  ------G-A---------------------A--A-G--G-------------------
 DPB28:  ------------------------------A--A-G--G-------------------
 DPB29:  ------G-A---------------------A--A-G--G-------------------
 DPB30:  ------------------------------A--A-G--G-------------------

             |-E-|                       |----F----|
```

The DNA sequences provided above are an important aspect of the present invention. Although only one strand of the sequence is shown, those of skill in the art recognize that the other strand of the sequence can be inferred from the information depicted above. This information enables the construction of the probes of the invention. Many illustrative probes of the invention are shown in the Examples below. However, suitable SSO probes for hybridization analysis of the DPB1 alleles will comprise (or be complementary to) certain polymorphic sequences. Six sets of illustrative probes of the invention are depicted below; each set is designed to distinguish between the polymorphisms in a specific region of the second exon of the HLA-DPB1 gene. The designation of the regions is as described above. The polymorphic residues encoded within the allelic variant in the segment to which a probe hybridizes is shown in one letter amino acid code (the dash means that the non-polymorphic prototype residue(s) present in those positions) to the left of the probe sequence. The probes span the regions encoding the polymorphic amino acid residues and are shown as having a length of about 18 nucleotides. Those sequences in the probe that encode the polymorphic amino acid residues, and thus must be included within a probe for detecting the alleles that encode the designated segment, are between the slash marks in the sequence. The DP alleles with which the probe will hybridize are shown to the right of the probe.

HLA-DPB1 SSO Probes

| Amino Acid Epitope | | Probe Sequence | DPB1 Alleles |
|---|---|---|---|
| **Segment A:** | | | |
| LF-G: | SEQ ID NO: 64 | TAC\CTTTTCCAGGG\ACGG | 2.1, 2.2, 4.1, 4.2, 5, 8, 16, 19, 21, 22, 24, 25, 26 |
| VY-L: | SEQ ID NO: 65 | TAC\GTGTACCAGTT\ACGG | 3, 6, 11, 13, 20, 23, 27, 30 |
| VY-G: | SEQ ID NO: 66 | TAC\GTGTACCAGGG\ACGG | 1, 15, 18 |
| VH-L: | SEQ ID NO: 67 | TAC\GTGCACCAGTT\ACGG | 9, 10, 14, 17, 28, 29 |
| **Segment B:** | | | |
| E-FA: | SEQ ID NO: 68 | CGG\GAGGAGTTCGC\GCGC | 4.1, 21, 22, 25 |
| E-FV: | SEQ ID NO: 69 | CGG\GAGGAGTTCGT\GCGC | 2.1, 3, 4.2, 6, 8, 9, 10, 14, 16, 17, 18, 19, 20, 24, 27, 28, 29 |
| E-LV: | SEQ ID NO: 70 | CGG\GAGGAGCTCGT\GCGC | 2.2, 5, 26, 30 |
| Q-YA: | SEQ ID NO: 71 | CGG\CAGGAGTACGC\GCGC | 11, 15 |
| E-YA: | SEQ ID NO: 72 | CGG\GAGGAGTACGC\GCGC | 1, 13, 23 |
| **Segment C:** | | | |
| AAE: | SEQ ID NO: 74 | CCTG\CTGCGGAG\TACTGG | 1, 4.1, 11, 13, 15, 22, 23, 25, 26 |
| DEE: | SEQ ID NO: 75 | CCTG\ATGAGGAG\TACTGG | 2.1, 4.2, 8, 10, 16, 18, 21 |
| EAE: | SEQ ID NO: 76 | CCTG\AGGCGGAG\TACTGG | 2.2, 5, 19, 28, 30 |
| DED: | SEQ ID NO: 77 | CCTG\ATGAGGAC\TACTGG | 3, 6, 9, 14, 17, 20, 27, 29 |
| **Segment D:** | | | |
| I-K: | SEQ ID NO: 78 | GAC\ATCCTGGAGGAGAA\G | 1, 4.1, 4.2, 5, 18, 23, 29 |
| I-E: | SEQ ID NO: 79 | GAC\ATCCTGGAGGAGGA\G | 2.1, 2.2, 8, 9, 10, 13, 16, 17, 19, 24, 25, 28, 30 |
| L-K: | SEQ ID NO: 80 | GAC\CTCCTGGAGGAGAA\G | 3, 14, 20, 21, 22, 26 |
| L-E: | SEQ ID NO: 81 | GAC\CTCCTGGAGGAGGA\G | 6, 27 |
| L-R: | SEQ ID NO: 82 | GAC\CTCCTGGAGGAGAG\G | 11, 15 |
| **Segment E:** | | | |
| M: | SEQ ID NO: 83 | GACAGG\ATG\TGCAGACAC | 2.1, 2.2, 4.1, 4.2, 5, 6, 11, 15, 16, 17, 18, 20, 21–26, 28, 30 |
| V: | SEQ ID NO: 84 | GACAGG\GTA\TGCAGACAC | 1, 3, 7, 8, 9, 10, 12, 14, 27, 29 |
| I: | SEQ ID NO: 150 | GACAGG\ATA\TGCAGACAC | 13, 19 |
| **Segment F:** | | | |
| GGPM: | SEQ ID NO: 85 | CTGG\GCGGGCCCA\TGACC | 2.1, 2.2, 4.1, 4.2, 24, 25 |
| DEAV: | SEQ ID NO: 86 | CTGG\ACGAGGCCG\TGACC | 1, 3, 5, 6, 8, 9, 10, 11, 13, 14, 16, 17, 19, 20, 22, 23, 27-30 |
| VGPM: | SEQ ID NO: 87 | CTGG\TCGGGCCCA\TGACC | 15, 18, 21, 26 |

Because the probes of the invention are single stranded for use in hybridization, it is important to note that merely because a probe is designed to hybridize with, for example, the coding strand, does not mean that an equally useful probe could not be designed that would hybridize to the complementary sequence present on the noncoding strand.

The sequence information provided above also relates to other important aspects of the invention. The preferred primers of the invention are designed to amplify many different DP alleles. In many instances, as demonstrated in the Examples below, such primers are very useful. However, those of skill in the art recognize that the DNA sequence information provided above can also be used to design primers that will enable allele specific amplification. Such allele-specific primers will only amplify a single allele or a certain subset of known alleles. For example, the "DEAV" probe of the invention can be used as one primer of a primer pair to provide for allele-specific amplification of "DEAV" positive DPB1 alleles.

The present invention also relates to kits useful for determining an individual's HLA DP genotype, comprising a panel of SSO probes and possibly suitable primers in accordance with the present invention. Said kits preferably consist of a multicontainer unit comprising the essential components for practicing the present method. For example, the kit can contain primers for PCR, as such primers are necessary in the preferred embodiment of the invention. These primers will amplify at least the DPB1 gene, and, when appropriate, for example in forensic analysis, primers can be included that also amplify the DPA1 gene. The kit must also contain SSO probes for at least the DPB1 gene, and, when appropriate, for the DPA1 gene as well. In some cases, the SSO probes may be fixed to an appropriate support membrane which is useful for the hybridization analysis. Other optional components that may be contained in containers within the kit include, for example, an agent to catalyze the synthesis of primer extension products, the substrate nucleoside triphosphates, means used to label (for example, an avidin-enzyme conjugate and enzyme substrate and chromogen if the label is biotin), and the appropriate buffers for PCR or hybridization reactions. In addition to the above components, the kit can also contain instructions for carrying out the present method.

A number of examples of the present invention, which are provided only for illustrative purposes and not to limit the scope of the invention, are presented below. Numerous embodiments of the invention within the scope of the claims that follow the examples will be apparent to those of ordinary skill in the art from reading the foregoing text and following examples. In the following examples, certain techniques are standard, unless specifically indicated otherwise. Such techniques include primed lymphocyte typing (PLT), which was performed essentially as described by Shaw et al., 1980, J. Exp. Med. 152:565-580. Generally, for lymphocyte priming, responder and stimulator cells were thawed, washed, and resuspended in RPMI-1640 medium supplemented with glutamine and antibiotics (complete media). Responding cells were mixed with irradiated stimulator cells in a ratio of 2:1, and the cell mixture was incubated for ten days at 37. The primed irradiated responder cells were co-cultured with irradiated stimulator cells in complete media. Alter 48 hours, $^3$H-thymidine was added to the culture. The cells were harvested 18 hours later, and tritium incorporation was evaluated by counting beta-emission.

DNA sequence analysis was performed as described by Maniatis et al., Molecular Cloning: A Laboratory Manual (New York, Cold Spring Harbor Laboratory, 1982). Generally, the sequences to be analyzed were cloned into an M13 cloning vector and analyzed by either the Maxam-Gilbert technique or by the dideoxy chain termination technique. Synthetic oligonucleotides, both primers and probes, were synthesized using commercially available instruments and techniques well known in the art. The skilled person is in a position to choose alternative reagents or instruments from other suppliers if necessary. Unless otherwise specified, percentages given below are on a wt/wt, vol/vol and wt/vol basis, respectively.

## Example 1

### Analysis of the DNA Sequences of HLA-DP Alleles

The DNA sequences of the variable second exons of a variety of alleles of the DPA1 gene and of the DPB1 gene were determined. The DNA samples used were chosen to represent as wide a spectrum of PLT defined DP alleles as possible. DNA was extracted from cell lines homozygous for the standard six DPw types, from cells exhibiting unusual typing reactions, and from cells exhibiting DP blank reactions. DNA extraction was by standard techniques, as described by Maniatis et al., in Molecular Cloning: A Laboratory Manual. The variable second exons of the DPA1 and the DPB1 genes were amplified by the PCR method, as described in Example 2, below. The amplified DNA sequences were cloned into an M13 derived vector, and the DNA sequences were determined by the chain termination method. The cell lines used, their DR serotypes, their PLT defined DPw types, and the DNA defined alleles they were found to contain are listed in tabular form below (blank spaces indicate data not determined).

| Cell Line | DR Type | DPw Type | DPB1 Alleles | DPA1 Alleles |
|---|---|---|---|---|
| CRK | 7 | 1* | 1, 11 | |
| RSO101 | w6 | 1, 3 | 1, 3 | |
| BMG | 4 | 1, 4 | 4.1 | |
| QBL | 3 | 2 | 2.2 | 1 |
| WJR | 2 | 2 | 2.1 | |
| PJM | 3 | 2, 3 | 2.1, 3 | |
| RMD | 5, w13 | 2, 4 | 2.1, 4.1 | |
| JMOS | 4, 5 | 2, 6 | 2.1, 6 | |
| SLE | w13 | 3 | 3 | 1 |
| COX | 3 | 3 | 3 | 1 |
| OPR | w8, w10 | 3, 4 | 3, 4.1 | |
| JAH | 4 | 3, 4 | 3, 4.1 | |
| MRI | 1, 2 | 3, 5 | 3, 5 | |
| HHK | w6 | 4 | 4.1 | |
| LB1 | 3, 7 | 4, MAS | 1, 4.2 | |
| APD | w6 | 4* | 2, 4 | |
| LUY | w8 | 1, 4 | 1, 4.1 | 1, 2 |
| WDV | w6 | 2, 4 | 2.1, 4.1 | |
| SMF | 2, w12 | 4, 5 | 4.1, 5 | |
| BCR | 4, w6 | 4, 6 | 4.1, 6 | |
| GTER | w8, 9 | 5 | 5 | |
| HAS | 4 | 5 | 5, 19 | |
| DKY | 9 | 5 | 2.1 | 1 |
| BIN40 | 4 | 3, 6 | 3, 6 | 1 |
| LG2 | 1 | ? | 4.1 | |
| PIAZ | 2.7 | | 8 | |
| TOK | 2 | | 9 | 2 |
| BM21 | w11 | | 10 | |
| VLA | 2, 3 | | 11 | |
| GBA | 1, 7 | | 11 | |
| CD1 | 3, 4 | 3, 6 | 10 | |
| CD2 | 7, (5) | 2, 4 | 4.2, 10 | |
| CD8 | 3, 7 | 4 | 4.1, 4.2 | |
| CD11 | | 2 | 2.1, 4.2 | |

DP "MAS" is provisional designation for a newly defined DP specificity related to the DPB4.2 allele. "CD" cell lines actually are samples from CD patients.

* - refers to cells with unusual DPw phenotypes.

As shown from the foregoing, the DNA analysis of the HLA-DP subtypes shows that specific sequences correlate with the known PLT-defined DPw typings, indicating that the polymorphic epitopes recognized by the primed T cells are on the DPB1 chain. For some DP types, e.g., DPw2 and DPw4, sequence analysis has revealed subtype variants. The variants for DPw2 have been designated DPB2.1 and DPB2.2; cells typed for PLT as DPw4 "new" (e.g., LB1) or DPw4* (e.g., APD) contain the rarer DPB4.2 subtype. The DPB4.2 subtype is more related by sequence to the DPB2.1 allele than to the DPB4.1 allele. Individual CD11 is PLT typed as DPw2, but contains the closely related DPB2.1 and DPB4.2 alleles.

The results above also show that unique DPB1 sequences correspond to the DPw1, DPw3, DPw5, and DPw6 specificities, and these alleles have been designated to reflect this correlation. A few exceptions are, however, that cell line DKY has been typed as DPw5, but contains the DPB2.1 allele, and that individual CD2 is PLT typed as DPw2, but contains the DPB4.2 and DPB10 alleles.

Example 2

Primers for the PCR Amplification of the DPA1 and DPB1 Genes

The DPA1 and DPB1 genes of some of the cells described in Example 1 were amplified by PCR. The primers used, which were synthetic, along with the region of the genes to which the primers bind, and which will act as templates for primer extension are shown below. As depicted in the table, the left side primers, GH98 and DB01, are from the upper strand, and direct DNA polymerase to extend rightward. The right side primers, GH99 and DB03, are from the lower strand, and direct synthesis leftward. Lower case letters indicate bases in the primer that are not complementary to the target genomic DNA (shown in the opposite strand). These changes in the primers incorporate restriction enzyme sites (BamHI or PstI) at the ends of the amplified DNA and facilitate cloning of the amplified DNA. The oligonucleotide primers GH98 and GH99, which are used for the amplification of the second exon of DPA1, amplify a 243 bp segment. The first two bp of the PCR product are from the intervening sequence which flanks the exon. The oligonucleotide primers DB01 and DB03 amplify a 294 bp segment of the second exon of DPB1. The left 13 bp and the right 17 bp of the product are from the intervening sequence. The genomic sequence to which the primers bind are listed in the Sequence Listing section under the following sequence identifiers:
Region to which GH98 binds - SEQ ID NO: 146
Region to which GH99 binds - SEQ ID NO: 147
Region to which DB01 binds - SEQ ID NO: 148
Region to which DB03 binds - SEQ ID NO: 149
The sequence of the region between the primer hybridization regions depends on the allele amplified. Allele sequences are provided in the Sequence Listing section under the sequence identifiers listed above.

**DPalpha Primers**

```
                                                 15
---------- GH98 ----------->  Phe Val Gln Thr His Arg Pro Thr ..
cGCGGAtCcTGTGTCAACTTATGCCGCG  TTT GTA CAG ACG CAT AGA CCA ACA ..
TCGCCTGGTACACAGTTGAATACGGCGC  AAA CAT GTC TGC GTA TCT GGT TGT ..


                     70
.. Leu Asn Asn Asn Leu Asn Thr Leu Ile
.. TTG AAC AAC AAC TTG AAT ACC TTG ATC CAGCGTTCCAACCACACTCAGGCCAC
.. AAC TTG TTG TTG AAC TTA TGG AAC TAG GTCGCAAGGTTGGTGTGAcgtCGGTc
                                         <-------- GH99 ---------
```

**DPbeta Primers**

```
                                 10                  15
------- DB01 -------> Leu Phe Gln Gly Arg Gln Glu Cys Tyr ..
CagggatCCGCAGAGAATTAC CTT TTC CAG GGA CGG CAG GAA TGC TAC ..
GGGGAGGGGCGTCTCTTAATG GAA AAG GTC CCT GCC GTC CTT ACG ATG ..


                   85                  90
.. Glu Leu Gly Gly Pro Met Thr Leu Gln
.. GAG CTG GGC GGG CCC ATG ACC CTG CAG CGCCGAGGTGAGTGAGGGCTTTGG
.. CTC GAC CCG CCC GGG TAC TGG GAC GTC GCGGCTCCACTCACTgaCGtcctg
                                      <-------- DB03 ---------
```

Hybridization of the primers and the synthesis of the elongated primer containing products were essentially as described in European Patent Application, Publication No. 258,017 and in the protocols provided by the manufacturer, Perkin Elmer (Norwalk, CT), of the Thermal Cycler used to perform the PCR. Amplification was for 28 cycles; however, more, i.e., 35, cycles can yield better results.

Two other primers for amplifying the second exon of DPB1 alleles, designated UG19 and UG21, have proven to be more efficient and specific than the primers discussed above. For amplification using UG19 and UG20, between 0.1 and 1 µg of genomic DNA is amplified in a 200 µl reaction containing 50 mM KCL, 10 mM Tris-HCL (pH 8.4), 1.5 mM $MgCl_2$, 100 µg/ml gelatin, 175 µM each dATP, dCTP, dGTP, and dTTP, 0.50 µM each amplification primer, and 5.0 units of Taq DNA polymerase. The amplification is carried out in a DNA Thermocycler (Perkin Elmer [Norwalk, CT]) using a two-step temperature cycle (denaturation: 95°C for 30 seconds; annealing and extension: 65°C for 30 seconds) for 30 cycles.

The sequences of the DPA1 and DPB1 primers discussed are provided below and in the sequence listing section.

| DPA1 Primers | | |
|---|---|---|
| Primer | Seq Id No. | Sequence |
| GH98 | SEQ ID NO: 140 | CGCGGATCCTGTGTCAACTTATGCCGC |
| GH99 | SEQ ID NO: 141 | CTGGCTGCAGTGTGGTTGGAACGC |

| DPB1 Primers | | |
|---|---|---|
| Primer | Seq Id No. | Sequence |
| DB01 | SEQ ID NO: 97 | CAGGGATCCGCAGAGAATTAC |
| DB03 | SEQ ID NO: 98 | GTCCTGCAGTCACTCACCTCGGCG |
| UG19 | SEQ ID NO: 144 | GCTGCAGGAGAGTGGCGCCTCCGCTCAT |
| UG21 | SEQ ID NO: 145 | CGGATCCGGCCCAAAGCCCTCACTC |

## Example 3

### SSO Probes for Hybridization Analysis of DPB1 Alleles

Illustrative probes of the invention referred to throughout the remainder of the Examples are described below in tabular form. In the Table, the probe designation, sequence identification number, probe sequence (5' to 3'), and the region and polymorphic amino acid sequence encoded in the region of the allele to which the probe hybridizes are provided. Probes are shown either as unlabeled or as having a $^{32}P$ or "X" label, where X represents HRP or biotin, as discussed in the Examples below. Where a probe sequence is indicated by an X followed by a probe designation, the sequence of the probe is identical to that of the probe designated after the X, except that the $^{32}P$ label has been replaced by an HRP label. As discussed in a later Example, the probes may be used in a reverse dot blot format in which the unlabeled probe is immobilized on a membrane and hybridized to the labeled PCR product. Probes shown below as unlabeled were designed for use in the reverse dot blot format; labeled versions could be used in other formats. Probes 154 and 155 each contain an inosine base which is denoted as "N" in the sequence.

SSOs for HLA-DPB1 Typing

| | Probe | Seq Id No. | Sequence |
|---|---|---|---|
| Region A | | | |
| LFQG | DB10 | SEQ ID NO: 99 | $^{32}$P-GAATTACCTTTTCCAGGGA |
| | DB27 | SEQ ID NO: 99 | X-DB10 |
| | DB70 | SEQ ID NO: 119 | X-GAATTACCTTTTCCAGGGAC |
| | DB136 | SEQ ID NO: 137 | CCGTCCCTGGAAAAGGTAATTC |
| VYQL | DB11 | SEQ ID NO: 100 | $^{32}$P-ATTACGTGTACCAGTTACG |
| | DB23 | SEQ ID NO: 111 | X-CGTAACTGGTACACGTAAT |
| | DB28 | SEQ ID NO: 100 | X-DB11 |
| | DB36 | SEQ ID NO: 111 | X-DB23 |
| | DB58 | SEQ ID NO: 114 | X-ATTACGTGTACCAGTTA |
| VYQG | DB12 | SEQ ID NO: 101 | $^{32}$P-CGTCCCTGGTACACGTAAT |
| | DB29 | SEQ ID NO: 101 | X-DB12 |
| | DB71 | SEQ ID NO: 120 | X-TTACGTGTACCTGGGAC |
| VHQL | DB22 | SEQ ID NO: 110 | $^{32}$P-ATTACGTGCACCAGTTACG |
| | DB35 | SEQ ID NO: 110 | X-DB22 |
| | DB117 | SEQ ID NO: 132 | ATTACGTGCACCAGTTAC |
| | DB118 | SEQ ID NO: 133 | ATTACGTGCACCAGTTA |
| | | | |
| Region B | | | |
| EEFARF | AB117 | SEQ ID NO: 151 | X-AGGAGTTCGCGCGCTT |
| EEFVRF | AB124 | SEQ ID NO: 152 | X-AGGAGTTCGTGCGCTT |
| EELVRF | AB119 | SEQ ID NO: 153 | X-AGGAGCTCGTGCGCTTC |
| QEYARF | AB120 | SEQ ID NO: 154 | X-CCGGCAGGAGTACGCGC |
| EEYARF | AB121 | SEQ ID NO: 155 | X-GAGGAGTACGCGCGCT |

| | | | |
|---|---|---|---|
| <u>Region C</u> | | | |
| AAE | DB13 | SEQ ID NO: 102 | $^{32}$P-CCTGCTGCGGAGTACTG |
| | DB30 | SEQ ID NO: 102 | X-DB13 |
| DEE | DB14 | SEQ ID NO: 103 | $^{32}$P-CAGTACTCCTCATCAGG |
| | DB31 | SEQ ID NO: 103 | X-DB14 |
| | DB75 | SEQ ID NO: 124 | X-CCTGATGAGGAGTACTG |
| | DB101 | SEQ ID NO: 131 | CCAGTACTCCTCATCAGGC |
| | DB121 | SEQ ID NO: 134 | CAGTACTCCTCATCAG |
| EAE | DB16 | SEQ ID NO: 104 | $^{32}$P-CAGTACTCCGCCTCAGG |
| | DB32 | SEQ ID NO: 104 | X-DB16 |
| | DB59 | SEQ ID NO: 115 | X-CCTGAGGCGGAGTACTG |
| DED | DB17 | SEQ ID NO: 105 | $^{32}$P-CCTGATGAGGACTACTG |
| | DB33 | SEQ ID NO: 105 | X-DB17 |
| | DB122 | SEQ ID NO: 135 | CTGATGAGGACTACTG |
| DEV | AB112 | SEQ ID NO: 92 | X-CCTGATGAGGTGTACTG |
| <u>Region D</u> | | | |
| I-K | DB18 | SEQ ID NO: 106 | $^{32}$P-GACATCCTGGAGGAGAAGC |
| | DB34 | SEQ ID NO: 106 | X-DB18 |
| | DB72 | SEQ ID NO: 121 | X-ACATCCTGGAGGAGAAGC |
| I-E | DB19 | SEQ ID NO: 107 | $^{32}$P-GCTCCTCCTCCAGGATGTC |
| | DB37 | SEQ ID NO: 107 | X-DB19 |
| | DB73 | SEQ ID NO: 122 | X-ACATCCTGGAGGAGGAGC |
| L-K | DB20 | SEQ ID NO: 108 | $^{32}$P-GACCTCCTGGAGGAGAAGC |
| | DB38 | SEQ ID NO: 108 | X-DB20 |
| | DB74 | SEQ ID NO: 123 | X-ACCTCCTGGAGGAGAAGC |
| L-E | DB21 | SEQ ID NO: 109 | $^{32}$P-GCTCCTCCTCCAGGAGGTC |
| | DB39 | SEQ ID NO: 109 | X-DB21 |
| | DB62 | SEQ ID NO: 116 | X-GACCTCCTGGAGGAGGAG |
| | DB92 | SEQ ID NO: 127 | X-GACCTCCTGGAGGAGGAGC |
| | DB155 | SEQ ID NO: 139 | GACCTCCTGGAGNGAGGAGC |
| L-R | DB63 | SEQ ID NO: 117 | X-GACCTCCTGGAGGAGAGG |
| | DB93 | SEQ ID NO: 128 | X-GACCTCCTGGAGGAGAGGC |
| | DB154 | SEQ ID NO: 138 | GACCTCCTGNGAGGAGAGGC |

| | | | |
|---|---|---|---|
| **Region E** | | | |
| M | AB96 | SEQ ID NO: 88 | TGTCTGCACATCCTGTCCG |
| V | AB97 | SEQ ID NO: 89 | TGTCTGCATACCCTGTCCG |
| I | AB98 | SEQ ID NO: 90 | CGGACAGGATATGCAGACA |
| **Region F** | | | |
| GGPM/VGPM | | | |
| | DB25 | SEQ ID NO: 112 | $^{32}$P-CTGCAGGGTCATGGGCCCCG |
| | DB40 | SEQ ID NO: 112 | X-DB25 |
| | DB64 | SEQ ID NO: 118 | X-CTGGTCGGGCCCATGACC |
| | DB76 | SEQ ID NO: 125 | X-CTGGGCGGGCCCATG |
| | DB94 | SEQ ID NO: 129 | X-AGCTGGGCGGGCCCATGAC |
| GGPM | AB122 | SEQ ID NO: 156 | X-CGAGCTGGGCGGGCCCA |
| VGPM | AB123 | SEQ ID NO: 157 | X-CGAGCTGGTCGGGCCCA |
| DEAV | DB26 | SEQ ID NO: 113 | $^{32}$P-CTGCAGGGTCACGGCCTCGTC |
| | DB41 | SEQ ID NO: 113 | X-DB26 |
| | DB95 | SEQ ID NO: 130 | X-AGCTGGACGAGGCCGTGAC |
| | DB77 | SEQ ID NO: 126 | X-CTGGACGAGGCCGTG |
| "ALL" | DB123 | SEQ ID NO: 136 | X-CGCTTCGACAGCGACGT |

With reference to the Table above, one should note that because DB28 cross-hybridizes with DB32, superior results can be obtained using probes DB58 and DB59 in place of DB28 and DB32, respectively. In addition, probe DB63 is preferred over DB93. Specific probe panels for use in an HLA-DP typing assay are discussed in the Examples which follow. The "ALL" probe, DB123, is specific for a nonvariable sequence just 3' of region B and hybridizes to all DRB1 allelic sequences. It is useful as a control for the amount of DRB1 DNA in the hybridization reaction.

Those of skill in the art recognize that depending on the type of label used and the hybridization format used, hybridization and wash conditions will differ. Although, in a preferred embodiment, the probes will be labeled nonisotopically (e.g., with HRP or biotin), some of the probes have been used with isotopic (e.g., $^{32}$P) labels. Hybridization and wash conditions for $^{32}$P-labeled and HRP-labeled probes used in a dot blot format are shown below, where such conditions have been empirically determined (see Bugawan et al., 1988, J. Immunol. 141(12):4024-4030, incorporated herein by reference). In the Table, the conditions referred to assume a hybridization solution composed of 5 x Denhardt's solution, 0.5% SDS, and the indicated amounts (i.e., 0.1 x, 3 x, 5 x) of SSPE. Five x Denhardt's solution contains 0.5 g Ficoll, 0.5 g polyvinylpyrrolidone, 0.5 g BSA (Pentax Fraction V) per 500 ml. The wash solution contains 0.1 x SSPE and 0.1% SDS (for HRP-labeled probes, 0.1% Triton X-100 was used in place of SDS); the wash step is carried out at the indicated temperature (celsius) for ten minutes in either a water bath or an air incubator. As described below, the use of tetramethyl ammonium chloride, or similar salts, can be used to allow for more uniform hybridization and wash conditions, a preferred condition when a number of probes are used in a panel to determine the types of DP alleles in a sample.

| Probe Hybridization/Wash Conditions | |
|---|---|
| Probe | Hybridization/Wash Conditions |
| DB10 | 5 x @ 50/42 $H_2O$ bath |
| DB27 | 5 x @ 50/42 air |
| DB11 | 3 x @ 42/42 $H_2O$ air |
| DB28 | 3 x @ 55/42 $H_2O$ bath |
| DB58 | 3-5 x @ 42/42 air |
| DB23 | 5 x @ 50/42 $H_2O$ bath |
| DB36 | 5 x @ 50/42 air |
| DB12 | 5 x @ 50/42 $H_2O$ bath |
| DB29 | 5 x @ 50/42 $H_2O$ bath |
| DB22 | 3 x @ 50/42 $H_2O$ bath |
| DB35 | 3 x @ 50/42 $H_2O$ bath |
| DB13 | 3 x @ 50/42 $H_2O$ bath |
| DB30 | 3 x @ 50/42 $H_2O$ bath |
| DB14 | 5 x @ 42/42 $H_2O$ bath |
| DB31 | 5 x @ 42/42 $H_2O$ bath |
| DB16 | 5 x @ 42/42 $H_2O$ bath |
| DB32 | 3-5 x @ 42/42 $H_2O$ bath |
| DB59 | 5 x @ 50/42 $H_2O$ bath |
| DB17 | 5 x @ 50/42 $H_2O$ bath |
| DB33 | 5 x @ 50/42 air |
| DB18 | 3 x @ 55/42 $H_2O$ bath |
| DB34 | 3 x @ 55/42 $H_2O$ bath |
| DB19 | 5 x @ 55/42 $H_2O$ bath |
| DB37 | 5 x @ 55/42 $H_2O$ bath |
| DB20 | 5 x @ 55/42 $H_2O$ bath |
| DB38 | 5 x @ 55/42 $H_2O$ bath |
| DB21 | 5 x @ 50/42 $H_2O$ bath |
| DB39 | 5 x @ 50/42 air |
| DB62 | 3 x @ 50/42 $H_2O$ bath |
| DB63 | 3 x @ 50/42 $H_2O$ bath |
| DB25 | 5 x @ 50/42 $H_2O$ bath |
| DB40 | 3 x @ 50/42 $H_2O$ bath |
| DB26 | 5 x @ 50/42 $H_2O$ bath |
| DB41 | 3 x @ 50/42 $H_2O$ bath |

When tetramethyl ammonium chloride (TMACL) is present in a hybridization solution, probe discrimination is based on probe length and not on the G, C, A, or T composition of the probe. Thus, by using TMACL in the hybridization solution, one can hybridize and wash many different probes of the same length at a single temperature.

A suitable hybridization solution for this purpose contains 3 M TMACL; 0.5% SDS; 10 mM Tris-HCl, pH = 7.5; and 0.1 mM EDTA. Hybridizations are carried out at 55 °C for 30 to 60 minutes for 19-mer probes DB27, DB28, DB29, DB35, DB34, DB37, DB38, and DB62; at 50 °C for 17-mer probes DB30, DB31, DB33, and DB59; and at 60 °C for DB40 and DB41. The wash solution is 3 M TMACL; 50 mM Tris-HCl, pH = 8; and 2 mM EDTA. The wash is carried out first at 37 °C for 20 minutes, then at the higher stringency temperature (the hybridization temperature) for 10 minutes.

Example 4

SSO Probes for Hybridization Analysis of DPA1 Alleles

Examples of suitable SSO probes for hybridization analysis of DPA1 alleles are shown below. Two sets of probes are illustrated; each set is designed to distinguish between the polymorphisms in a specific segment of the second exon of the HLA-DPA1 gene. ASO1 and ASO2 bind to the DPA1 allele at the region containing the polymorphic segments containing methionine (M) (amino acid 31) and glutamine (Q) (amino acid 50), respectively. ASO3 and ASO4 bind to the DPA2 allele to the region containing the polymorphic segments which contain glutamine (amino acid 31) and arginine (R) (amino acid 50), respectively. ASO2 and ASO4 distinguish the polymorphic segment at position 50 containing glutamine from that containing arginine. The probes span the regions encoding these polymorphic amino acid residues. Hybridization using these probes is usually carried out in a solution containing 5 x SSPE, 5 x Denhardt's, and 0.5% SDS, for at least 1 hour at 42°C. Also shown are the washing conditions (temperature in degrees celsius) for use with the probes. The probe sequences are shown 5' to 3'.

| HLA-DPA1 SSO Probes | | | |
|---|---|---|---|
| Probe | Seq Id No. | Sequence | Wash Conditions |
| ASO1 | SEQ ID NO: 93 | AGATGAGATGTTCTATG | 2 x SSPE, 0.1% SDS, 42 |
| ASO2 | SEQ ID NO: 94 | GTTTGGCCAAGCCTTTT | 2 x SSPE, 0.1% SDS, 50 |
| ASO3 | SEQ ID NO: 95 | AGATGAGCAGTTCTATG | 2 x SSPE, 0.1% SDS, 50 |
| ASO4 | SEQ ID NO: 96 | GTTTGGCCGAGCCTTTT | 2 x SSPE, 0.1% SDS, 55 |

## Example 5

### Analysis of Amplified DPB1 Sequences by Hybridization with SSO Probes

PCR amplified DPB1 sequences from 24 HTCs (homozygous typing cells) were analyzed in a dot blot format with a panel (n = 9) of $^{32}$P-labeled SSO probes, and the DPB1 type was inferred from the pattern of probe binding. The extraction of DNA from the cells was as described in Example 1. The target regions of the cellular genome, i.e., the second exon of the DPB1 genes, were amplified by the PCR technique using primers DB01 and DB03 as described in Example 2, except that the DNA was from the cells listed in tabular form above. At the time of this study, only a subset of the currently known alleles were known; additional alleles have since been discovered using the methods of the present invention.

The amplified DNAs were dot blotted onto a filter; a separate filter containing the panel of samples was prepared for analysis by hybridization with each SSO probe. To dot blot the samples, five microliters of each amplified sample were denatured by diluting the sample with 195 microliters of a solution containing 0.4 N NaOH and 25 mM EDTA and spotted onto 9 replicate Genatran 45 (Plasco, Woburn, Massachusetts) nylon filters by first wetting the filters with water, placing them in a Bio-dot (Bio-Rad, Richmond, CA) apparatus for preparing dot blots, applying the samples, and rinsing each well with 0.4 ml of 20 x SSPE (3.6 M NaCl, 200 mM $NaH_2PO_4$, and 20 mM EDTA). The filters were removed, rinsed in 2 x SSPE, and baked for 30 minutes at 80°C in a vacuum oven.

The samples on the filters were hybridized with SSO probes of the invention. Hybridization was with 0.25 to 0.5 pmoles of probe in 2 to 5 ml of hybridization solution. Hybridization and wash conditions were as described in tabular form in Example 3. The results of this DPB1 typing are shown below, which also shows the probe with which the samples on the filter were hybridized and the encoded amino acid sequence detected by the probe.

## DPbeta Oligonucleotide Probes

| | CELL LINE | HLA DR | HLA DPw | A: DP10 LFQG 4,2,5, 7,8 | A: DB11 VYQL 6,3,11 | A: DB12 VYQG 1 | C: DB13 AAE 4.1,7, 1,11 | C: DB14 DEE 4.2,8, 2.1,10 | C: DB17 DED 6,3,9, 12 | D: DB18 I-K 4,5,7, 1 | D: DB19 I-E 2,8,9, 10,12 | D: DB20 L-K 3 | DPbeta Allele type |
|---|---|---|---|---|---|---|---|---|---|---|---|---|---|
| *1 | COX | 3 | 3 | - | + | - | - | - | + | - | - | + | 3 |
| 2 | 950 | 3 | 1 | - | - | + | + | - | - | + | - | - | 1 |
| * 3 | BM21 | 11 | blank | - | +/-c | - | - | + | - | - | + | - | 10 |
| 4 | BM16 | 12 | 2 | + | - | - | - | + | - | - | + | - | 2.1 (or 8) |
| 5 | BR1G | | 2 | + | - | - | - | + | - | + | + | - | 2.1/4.2 or 4.2/8 |
| 6 | JVM | 5 | 2 | + | - | - | - | + | - | - | + | - | 2.1 (or 8) |
| 7 | WT46 | 6 | 2 | + | - | - | - | + | - | - | + | - | 2.1 (or 8) |
| 8 | MANN | 7 | 2 | + | - | - | - | + | - | - | + | - | 2.1 (or 8) |
| 9 | J640 | 4 | 3 | - | + | - | - | - | + | - | - | + | 3 |
| 10 | SLE | 13 | 3 | - | + | - | - | - | + | - | - | + | 3 |
| 11 | JMF | 7 | 4 | - | + | - | - | - | + | - | - | + | 3 |
| 12 | BGE | 2 | 4 | + | - | - | + | - | - | + | - | - | 4.1 |
| 13 | BM14 | 4 | 4 | + | - | - | + | - | - | + | - | - | 4.1 |
| 14 | BM92 | 4 | 4 | + | -/+d | - | - | + | - | + | - | - | 4.2 |
| 15 | TS10 | 4 | 4 | + | - | - | + | - | - | + | - | - | 4.1 |
| 16 | RLO | 4 | 4 | + | - | - | + | - | - | + | - | - | 4.1 |
| *17 | APD | 6 | 4 | + | - | - | - | + | - | + | - | - | 4.2 |
| 18 | BUP | 7 | 4 | + | - | - | + | + | - | + | - | - | 4.1/4.2 |
| 19 | MMR | | 4 | + | - | - | + | - | - | + | - | - | 4.1 |
| *20 | LUY | 8 | 4/1 | + | - | + | + | - | - | + | - | - | 1/4.1 |
| 21 | YOS | 4 | 4 | + | - | - | - | + | - | + | -/+d | - | 4.2 |
| *22 | DKY | 9 | 5 | + | - | - | - | + | - | - | + | - | 2.1 (or 8) |
| 23 | HAS | 4 | 5 | + | - | - | - | - | - | + | -/+d | - | 5 |
| *24 | TOK | blank | - | -/+c | - | - | - | + | - | + | - | 9 | |

* Cell line DPbeta type confirmed by DNA sequencing. +/- designates weak signal. Superscript c indicates the presence of an additional polymorphic sequence in region A encoding the residues VHYL, which cross-hybridizes with the DB11 probe (no cross-hybridization occurs with probe DB22). Superscript d represents cross-hybridizing due to the ability of the DB11 probe to bind to sequences recognized by the DB10 probe (cell line BM92) or due to the ability of the DB19 probe to bind to sequences recognized by the DB18 probe (cell lines YOS and HAS).

The determination of DPB1 types of the cells based upon the hybridization analysis with the SSO probes has been discussed above. To determine the DPB1 type of a sample, the binding of the probes to the sample DNA was examined. The alleles present were inferred from the pattern of probe binding. For example, sample 1 formed hybrids with SSO probes DB11, DB17, and DB20. The amino acids encoded by DB11, DB17, and DB20 are VYQL, DED, and LEEK, respectively. An examination of segments A, C, and D of the DPB1 allelic amino acid sequences shows that the sequence VYQL is present in DPB3, DPB6, DPB11, and DPB13; the sequence DED is present in DPB17, DPB14, DPB12, DPB9, DPB6, and DPB3; the sequence LEEK (L-K) is present in DPB14 and DPB3. The only allele (known at the time of the study) which contains the three sequences which hybridize with the probe is DPB3. Thus, the DP type of sample 1 based on SSO typing is DPB3.

The DPB1 types of the other samples were inferred by the same type of analysis, and the determined types are depicted above. In the depiction, the asterisk (*) indicates cells where the DPB1 genotype was also determined by sequence analysis. The DPB1 type of the cell lines COX (formerly w1 -> w3) and BM21 (formerly w1 -> blank) have recently been changed to the type indicated. The symbol +/- refers to a weak signal obtained with a probe. In some cases (BM21 and TOK), this weak signal reflects the presence of an additional polymorphic sequence in region A encoding the amino acid residues VHQL, which cross-hybridizes to the DB11 probe. The sequence can be more conveniently typed with region A probe DB22. For other cell lines (BM92), there is apparently a background cross-hybridization of the DB11 probe to the sequences recognized by the DB10 probe. In similar fashion, cross-hybridization signals can occur with the DB19 probe on sequences complementary to the DB18 probe.

The panel of SSOs used in this Example detects variation at only 3 of the 5 polymorphic regions and does not detect all allelic variants at these 3 regions. The typing system using the procedure of this example is simple and unequivocal for HTCs, but given the patchwork pattern of polymorphism, can give rise to ambiguous typing for heterozygous individuals if the hybridization pattern of the various probes can be interpreted as more than a unique pair of alleles. This ambiguity arises from the many different combinations of the DPB1 sequence variants that constitute the different DPB1 alleles. However, with the use of the additional SSO probes provided by the invention, which additional probes span the remaining polymorphic regions, and, possibly, allele-specific amplification as described above, unambiguous typings can be obtained for heterozygous individuals.

## Example 6

### HLA-DP Typing of Coeliac Disease Patients by DNA Sequence Analysis of PCR Amplified Target Regions

The cells of four patients with coeliac disease (CD) were PLT-typed, and the DNA sequences of the second exon of the DPB1 gene determined as described in Example 1. The CD diagnosis was based on clinical symptomology.

The results of the analysis obtained by DNA sequencing of the DPB1 (second exon) of the CD cells has been discussed above. From these results, one observes that, when CD cells are compared to non-CD cells, there is an apparent increase in the frequency of the DPB4.2 allele. In addition, the DPB10 allele sequence is present in two independent CD patients yet observed in only one of the 30 non-CD cell lines.

## Example 7

### HLA-DP Typing of Coeliac Disease Patients by SSO Probe Hybridization Analysis

The cells of 19 patients with CD and of 43 non-CD controls were analyzed for HLA-DP type by SSO probe hybridization analysis. The diagnosis of CD was based on clinical symptomology; the CD patients as well as control individuals were all from Italy. DNA extraction was as described in Example 1. PCR amplification of the samples was as described in Example 2. Analysis of the amplified sequences was as described in Example 4.

The results of the analysis show that there was a significant increase in the DPB4.2 allele in CD patients as compared to non-CD controls; this allele was present in 12 of 19 CD patients, but only 3 of 43 control patients. The DPB4.2 and DPB3 alleles were present in 17 of 19 CD patients and in 15 of 43 controls. The genotype DPB4.1/4.2 was present in 10 of 19 CD patients and in only 1 of 43 controls.

## Example 8

### HLA-DP Typing of Forensic Samples

Samples which contain genomic nucleic acids of the suspect individual are obtained. The target regions of the genome, i.e., the regions containing the second exon of the DPA1 and DPB1 genes, are amplified by the PCR technique, as described in Example 2, except that the nucleic acids from the suspect replace those of the cells in the Example, and except that the amplified samples contain a $^{32}P$-label. The amplified

sample is hybridized with the probes of the invention, which have been dot blotted onto the same filter, and which have been immobilized on the filter by poly-dT tails. The techniques for preparing the immobilized sequence-specific probes are described in International Patent Application, Publication No. WO 89/11548. The hybridization and washing conditions for the filter allow only perfectly matched hybrids to remain in duplex state. The filters are examined to determine which probes form hybrids with the labeled sample.

A sample which is to be compared with that obtained from the suspect is examined for DP type by the same procedure, i.e., by PCR amplification and hybridization with immobilized SSO probes. The pattern of binding to the SSO probes of the sample from the suspect and of the comparison sample is examined to determine the similarity or difference in the hybridization patterns.

Example 9

HLA-DP Typing Using SSO Probes Labeled with Horseradish Peroxidase

A panel of cells from 39 individuals was typed for DPB1 alleles using SSO probes for the second exon variants. The probes were labeled with horseradish peroxidase (HRP), and hybrids detected in a dot blot format.

The cells analyzed were from fourteen IDDM patients, five DR3 control non-IDDM patients, and nineteen HTCs which typed by PLT as DP blank. IDDM patients were identified by clinical symptomology. DNA was isolated from the cells as described in Example 1. The target region, i.e., the second exon of the DPB1 genes, was amplified using the PCR technique in 200 microliters of reaction mixture that contained 50 mM Tris-HCl, pH 8.3; 2.5 mM $MgCl_2$; 100 micrograms/ml gelatin; 0.75 mM of each of the four deoxynucleoside triphosphates; the primers DB01 and DB03; and Taq polymerase. The amplification temperature cycling profile was the following: heating for 30 seconds to 94°C followed by incubation at that temperature for 30 seconds; cooling for 1 minute to 55°C followed by incubation at that temperature for 30 seconds; heating to 72°C for 30 seconds followed by incubation at that temperature for 45 seconds. This cycling was repeated for 42 cycles. After amplification, the reaction mixtures were sampled and monitored by gel electrophoresis on gels containing 3% Nusieve, 1% Agarose to determine if all the DNA amounts were comparable.

Filters containing the dot blotted samples were prepared by blotting 150 microliters/dot of denatured amplified DNA on Genatran nylon membranes and UV treating the filters containing the samples for 5 minutes. The latter treatment is to fix the sample to the membranes. The amplified DNAs were denatured by treating 5 microliters of the PCR reaction mixture in a total volume of 150 microliters containing 0.4 N NaOH and 25 mM EDTA. Eight replicate filters were prepared for hybridization with HRP labeled ASO probes.

Prior to hybridization, the sample containing filters were incubated for 15 minutes in pre-hybridization solution (1 x SSPE, 5 x Denhardt's solution, 1% Triton X-100) without probe. In the pre-hybridization solution, Triton X-100 was used in place of SDS. Hybridization was in the same solution, which additionally contained 1 picomole probe/ml. Each filter was incubated for 40 minutes with 2.5 ml of hybridization solution containing one of the HRP labeled probes. The probes used were DB27, DB28, DB29, DB30, DB31, DB32, DB33, and DB35. The probes and hybridization conditions are listed in tabular form in Example 3. After hybridization, the filters were washed as stated in Example 3, under suitably stringent conditions, i.e., in 0.1 x SSPE, 0.1% Triton X-100 for 10 minutes at 42°C.

The HRP-labeled SSO probes were prepared essentially by the methods disclosed in U.S. Patent Nos. 4,962,029 and 4,914,210 and in corresponding International Patent Application, Publication Nos. WO 89/02932 and WO 89/02931. See also, Levenson and Chang, "Nonisotopically Labelled Probes and Primers" in PCR Protocols pages 99-112, Academic Press, 1990, M. Innis, ed. These methods essentially involve derivatizing the nucleic acid probe using a linear linking molecule comprising a hydrophilic polymer chain (e.g., polyoxyethylene) having a phosphoramidite moiety at one end and a protected or unprotected sulfhydryl moiety at the other end. The phosphoramidite moiety couples to the nucleic acid probe by reactions well known in the art (e.g., Beaucage et al., 1988, Tetrahedron Lett. 22: 1859-1862), while the sulfhydryl group is free to form disulfide or other covalent bonds with the protein, e.g., HRP. In U.S. Patent No. 4,962,029 and International Patent Application, Publication No. WO 89/02932, the HRP is conjugated to the linking molecule through an N-maleimido-6-aminocaproyl group. The label is prepared by esterifying N-maleimido-6-aminocaproic acid with sodium 4-hydroxy-3-nitrobenzene sulfonate in the presence of one equivalent of dicyclohexylcarbodiimide in dimethylformamide. After purification, the product is added to phosphate buffer containing HRP at a ratio of 1:8 HRP to ester. The oligonucleotide probe is synthesized in a DNA synthesizer, and the linking molecule having the structure $(C_6H_5)_3CS\text{-}(CH_2CH_2O)_4\text{-}P(CH_2CH_2CN)[N\text{-}(i\text{-}PR)_2]$ attached using phosphoramidite synthesis conditions. The trityl group is removed, and the HRP derivative and probe derivative are mixed together and allowed to react to form the labeled probe. A biotin-labeled probe or primer may be prepared by similar methods.

Samples which contained hybridized probe were detected using a color development reaction, as described in Sheldon et al., 1986 Proc. Natl. Acad. Sci. USA 83:9085-9089, which utilizes TMB/$H_2O_2$. The detection system is described in International Patent Application, Publication No. WO 89/11548. The HLA-DP genotypes of the amplified DNA samples were readily apparent from the filters.

## Example 10

## HLA-DPB1 Typing with HRP Labeled SSO Probes

### A. PCR Amplification

DPB1 typing can utilize as many as or more than 14 SSO probes (sequence specific oligonucleotides), so amplification is carried out on 0.5 to two micrograms of DNA, in 200 microliters of reaction volume, if DNA is not limiting. Lower amounts of DNA, i.e., 100 ng, can be amplified, but more cycles of amplification should be performed with such samples, i.e., 45 cycles.

The PCR reaction is started by mixing the following by vortexing for 1 to 2 seconds.

| | |
|---|---|
| DNA | 0.5 to 2 mg |
| 10 x Taq buffer | 20 µl |
| 100 mM dNTPs | 1.5 µl |
| DPB1 primer [10 mM UG19 or DB01] | 10 µl |
| DPB1 primer [10 mM UG21 or DB03] | 10 µl |
| Taq polymerase 5 U/ml | 1.2 µl |

Glass-distilled $H_2O$ is added to achieve a final volume of 200 µl. 10 X Taq salts are 500 mM KCl; 100 mM Tris, pH 8.3; 15 mM $MgCl_2$; and 1 mg/ml gelatin. Negative controls (i.e., no DNA) should be included in each PCR run. Typically, 30-35 cycles of amplification in a Perkin Elmer (Norwalk, CT) DNA Thermal Cycler are sufficient. The cycles are designed to denature at 96°C for 30 seconds, and anneal and extend at 65°C for 30 seconds. If primer pair DB01/DB03 is used, annealing is at 55°C for 30 seconds and extension is at 72°C for 30 seconds. Analytical gels can be used to check PCRs and to quantitate amount of DNA to be used for dot-blots.

### B. Dot blot

Typically, 5 µl of the amplified DNA contains approximately 200 ng, more than enough for a single dot blot. Remember, however, that as many as or more than 14 dots may be required, i.e., about 70 µl of the amplification reaction would then be used in preparing the dot blots. For each 5 µl of amplification reaction, 50 µl of 0.4 N NaOH and 25 mM EDTA are added to the DNA. Five minutes is sufficient to complete denaturation of DNA. The Genatran membrane is first wetted in 2 x SSPE, and then the 55 µl of denatured DNA are loaded into the dot blot apparatus. The membrane is rinsed in 2 x SSPE, and the DNA is fixed to the membrane with exposure to UV light for five minutes, i.e., by a 55 mJ/$cm^2$ exposure in a Stratalinker 1800™ UV light box, marketed by Stratagene.

### C. Hybridization

The membrane is again wetted in 2 x SSPE, and about 5 ml of hybridization solution per 8 x 12 cm membrane (size of dot blot apparatus) are added. About 1 to 1.5 picomoles of HRP probe are added per ml hybridization solution, and the probes are allowed to hybridize for at least one hour. Hybridization solution is SSPE (as indicated above), 5 x Denhardt's, and 1% Triton X-100. The membranes are then washed with 0.1 x SSPE and 0.2% Triton X-100 for ten minutes. Otherwise, hybridization and wash conditions were as described in Example 3. The probes used are DB27, DB29, DB30, DB31, DB33, DB34, DB35, DB37, DB38, DB40, DB41, DB58, DB59, DB62, and DB63.

D. Detection

The following steps for detection of probes are done at room temperature with moderate shaking and just enough solution to cover the membrane completely, as is described in Bugawan et al., 1988, Bio/Technology 6:943-947, incorporated herein by reference. The detection is carried out by a 5 minute incubation of the membrane with Buffer B, a 5 minute wash with Buffer C, and 10 minutes incubation under light exclusion with Buffer C and TMB (48 ml of Buffer C and 2.5 ml of 2 mg/ml TMB). Buffer B is 100 mM NaCl, 1 M urea, 5% Triton X-100, and 1 % dextran sulfate. Buffer C is 100 mM sodium citrate, pH 5.0. TMB is 3, 3', 5, 5'-tetramethylbenzidine. About 23 µl of 3% $H_2O_2$ are added to 50.5 ml of Buffer C/TMB; the resulting solution is used to develop the color on the membranes (color comes up within 1 to 15 minutes) under light-excluding conditions. The color development is stopped by washing the filters in $H_2O$ with a small amount of Buffer C. The Buffer C wash is repeated twice for 30 minutes. Pictures of the membrane are taken and the membrane is stored in Buffer C under light exclusion.

The methods described herein, as well as the SSO probes and primers, and kits containing them, are useful for the accurate, relatively simple, and economic determination of an individual's HLA-DP genotype. Accurate DP typing will prove important in several medical applications. For example, accurate HLA-DP matching of donors and recipients may be helpful in the prevention of allograft rejection and in the prevention of graft versus host disease. Because certain HLA-DP genotypes appear to be linked to certain autoimmune diseases, including, for example, coeliac disease, pauciarticular JRA, and IDDM, HLA-DP DNA typing may be useful in early diagnosis of the disease, prior to manifestation of full clinical symptoms.

Accurate HLA-DP typing is useful in forensic medicine. For example, it provides evidence as to whether a sample which contains genomic nucleic acids, for example, blood, hair, or semen is derived from a suspect individual. It is also useful in determining an individual's paternity or maternity. The latter is of particular importance in analyzing historical samples.

## Example 11

15-Probe DPB1 Typing Assay Dot Blot and Reverse Dot Blot Formats

Both dot blot and reverse dot blot hybridization protocols have been described above. Described here are DPB1 typing assays using each of the hybridization protocols. These assays use 15 probes - 4 for region A, 4 for region C, 5 for region D, and 2 for region F. Both protocols have been used successfully for typing at the DPB1 locus as described in Bugawan et al., 1990, Immunogenetics 32:231-241, incorporated herein by reference.

A. Dot Blot

Amplification is performed using primers UG19 and UG21 using the amplification protocol described for these primers in Example 2.

In the dot blot format, the amplified DNA is immobilized on a membrane. Approximately 100 ng of amplified DNA is denatured in a solution of 45 µl of 0.4 NaOH and 25 mM EDTA for 10 minutes at room temperature. The membrane (Genetrans-45 [Plasco, Woburn, Massachusetts] or Biodyne [Pall, Glen Cove, New York]) is pre-wet in 2 x saline-sodium phosphate-EDTA (SSPE) or 10 mM Tris, 0.1 mM EDTA. Then, 50 µl of the denatured DNA sample is applied to the membrane using a dot blot apparatus (Biodot, BioRad, Richmond, California). The DNA is ultraviolet (UV) crosslinked to the membrane using a Stratalinker (Stratagene, La Jolla, California) at 50 $mJ/cm^2$. Unbound DNA is removed by briefly rinsing the membrane in the same solution used to pre-wet the membrane.

Hybridization is carried out essentially as described in the Examples above. Probes and hybridization conditions are shown below. Two sets of probes and the hybridization and wash conditions are shown. The first set uses a hybridization solution of SSPE (at the indicated concentration) and 0.5% sodium dodecyl sulfate (SDS). Hybridization is carried out in a shaking water bath (except where noted) at the indicated temperature (celsius) for 30-60 minutes with 1 pmol HRP labeled probe per ml hybridization solution (8 ml hybridization solution are used per 96-sample filter). Unbound probe is then removed by washing the filters for 10 minutes in 0.1 x SSPE plus 0.1% SDS at the indicated temperature.

The second set of probes and hybridization conditions are for hybridization using TAMCl (described in Example 3). Hybridization is carried out at the indicated temperature in 3 M TAMCl, 0.5% SDS, 10 mM Tris (pH 7.2), and 0.1 mM EDTA as described above. Unbound probe is removed by washing the filters in 3 M TAMCl, 50 mM Tris (pH 7.2), and 2 mM EDTA for 10 minutes at 37°C and then for 10 minutes at the indicated stringent temperature.

In the table below, probes in parentheses are probes designed to work under the hybridization conditions for TAMCl. Where no second probe is shown, the indicated probe is used for both SSPE and TAMCl hybridization conditions. The sequences of all DBP1 probes are provided in the Examples above and in the Sequence Listing section. Also included is one probe (DB123, SEQ ID NO: 136, 5'CGCTTCGACAGCGACGT) which is specific for a nonvariable sequence just 3' of region B and hybridizes to all DPB1 allelic sequences. This "All" probe is used to control for the amount of amplified DPB1 DNA in the hybridization reaction.

**Probes for Dot Blot Hybridization**

| Probe | SSPE Hybridization Concentration | Hybridization/Wash Temperature SSPE (°C) | TAMCl (°C) |
|---|---|---|---|
| **Region A** | | | |
| DB10 | 5 x | 50/42 | 55/58 |
| DB11(DB58)* | 3 x | 42/42 | 52/58 |
| DB12 | 5 x | 50/42 | 55/58 |
| DB22 (DB117) | 3 x | 50/42 | 55/58 |
| **Region C** | | | |
| DB13 | 3 x | 50/42 | 55/58 |
| DB14 (DB121) | 5 x | 42/42 | 55/58 |
| DB59 | 5 x | 50/42 | 55/58 |
| DB17 (DB122)* | 5 x | 50/42 | 55/58 |
| **Region D** | | | |
| DB18 (DB72) | 3 x | 55/42 | 55/58 |
| DB19 (DB73) | 5 x | 55/42 | 55/60 |
| DB20 | 5 x | 55/42 | 55/58 |
| DB62 | 3 x | 50/42 | 52/58 |
| DB63 | 3 x | 50/42 | 55/58 |
| **Region F** | | | |
| DB40 | 3 x | 50/42 | 55/58 |
| DB41 | 3 x | 50/42 | 55/58 |
| **All** | | | |
| DB123 | 3 x | 50/42 | 55/58 |

* An air incubator is used for these probes instead of a shaking water bath.

Hybridization ofthe HRP-labeled probe to the immobilized DNA is detected by using the colorless soluble substrate, tetramethyl benzidine (TMB, Fluka, Ron Kon Koma, New York), which is converted to a blue precipitate by HRP in the presence of hydrogen peroxide. The detection is carried out at room temperature with moderate shaking as follows. After washing, the membranes are incubated for 30 minutes in 1 x Dulbecco's phosphate-buffered saline and then transferred to buffer C (100 mM sodium citrate, pH 5) plus 0.1 mg/ml TMB. TMB is precipitated by the immediate addition of hydrogen peroxide to a final concentration of 0.0015% and appears as a blue precipitate in 1 to 5 minutes. The reaction is stopped by transferring the membranes to 0.01 x buffer C. For a permanent record, the membranes can be photographed. The precipates are stable for over 2 months if stored individually in the dark with buffer C. Alternatively, hybridization of the HRP-labeled probes can be detected using the highly sensitive, commercially available ECL Gene Detection System Kit (Amersham, Arlington Heights, Illinois).

B. Reverse Dot Blot

In the reverse dot blot format, the probes are immobilized on a membrane and hybridized to the biotinylated amplified DNA. The protocols used are essentially as described in Saiki et al., 1989, Proc. Natl. Acad. Sci. USA 86:6215-6219, incorporated herein by reference.

Amplification is performed as for the dot blot described above with the exception that each primer contains a single biotin molecule attached at the 5' end of the oligonucleotide. The 15 probes, shown below, are given poly-T tails using either terminal deoxyribonucleotidyltransferase or chemical synthesis and bound to the filters. Due to their length, the tails are preferentially bound to the membrane, leaving the oligonucleotide probe free to hybridize. Unbound probe is removed by washing the membranes for at least 30 minutes in 1 x SSPE, 0.5% SDS at 50 °C.

The 15 probes used are listed below grouped according to the region of the gene to which they hybridize. The specific amino acid epitopes detected are the same epitopes detected by the 15 probes used in the dot blot format described above. The sequence of the hybridization region of each probe is provided in the Examples above and in the Sequence Listing section. The "All" probe described above also is included here.

| | Reverse Dot Blot Probes |
|---|---|
| Region A | DB136, DB36, DB12, DB118 |
| Region C | DB13, DB101, DB59, DB17 |
| Region D | DB72, DB73, DB74, DB155, DB154 |
| Region F | DB94, DB95 |
| All | DB123 |

For hybridization, the biotinylated amplified DNA is denatured at 95 °C for 5 minutes and then cooled on ice. Fifty µl of denatured DNA is added to the filters in 2.5 ml pre-warmed (50 °C) hybridization solution (1 x SSPE, 0.5% SDS) along with 70 µl of 20 mg/ml streptavidin-HRP conjugate (Amplitype™, Hoffmann-La Roche Inc., Nutley, New Jersey). Hybridization is carried out for 30 minutes at 50 °C in a shaking water bath. Unbound probe is removed by washing the filters for 10 minutes in 0.25 x SSPE, 0.1% SDS at 42 °C in a shaking water bath. Hybridization is detected as in the dot blot assay.

Example 12

25 Probe DPB1 Typing Assay

A dot blot DPB1 typing assay essentially as described in Example 11 but modified to include five additional probes for the five different epitopes at amino acids 33-36 (Region B), three additional probes for the three variable amino acids at position 76 (region E), and two additional probes to differentiate the GGPM and VGPM epitopes at amino acids 84-87 (Region F) has also been designed. The set of probes, shown below, differs from those described in Example 11; however, the variable regions probed and the sequences detected are the same with the addition of the five probes for Region B, the three probes for Region E, and the two probes for Region F noted above. As in Example 11, the "All" probe, DB123, is included with the set of probes as a control. The sequences are provided in the Examples, above, and in the Sequence Listing section.

Hybridization is carried out essentially as described in the Example 11, above. Probe hybridization and wash conditions are shown in the table below. A hybridization solution of SSPE at the indicated concentration and 0.5% sodium dodecyl sulfate (SDS) is used. Hybridization is carried out in a shaking water bath, except where noted, at the indicated temperature (celsius) for 20-60 minutes with 2 pmol HRP labeled probe per ml hybridization solution (8 ml hybridization solution are used per 96-sample filter). Unbound probe is then removed by washing the filters in 0.1 x SSPE plus 0.1% SDS using the below indicated conditions.

| Region | Probes | Epitoues | Hybridization | | Wash Temp. | Wash Solution | Wash Time |
|---|---|---|---|---|---|---|---|
| | | | Temp. | Solution | | | |
| A | DB27 | LFQG | 50 °C | 5 X | 40 °C (air) | 0.1 X | 10 min. |
| A | DB29 | VYQG | 50 °C | 5 X | 42 °C | 0.1 X | 10 min. |
| A | DB35 | VHQL | 50 °C | 2 X | 42 °C | 0.1 X | 15 min. |
| A | DB36 | VYQL | 42 °C | 2 X | 42 °C | 0.1 X | 15 min. |
| B | AB117 | EEFARF | 55 °C | 5 X | 50 °C | 0.1 X | 15 min. |
| B | AB119 | EELVRF | 50 °C | 5 X | 50 °C | 0.1 X | 12 min. |
| B | AB120 | QEYARF | 50 °C | 3 X | 50 °C | 0.1 X | 15 min. |
| B | AB121 | EEYARF | 55 °C | 1 X | 50 °C | 0.1 X | 12 min. |
| B | AB124 | EEFVRF | 55 °C | 1 X | 42 °C | 0.1 X | 15 min. |
| C | DB30 | AAE | 50 °C | 3 X | 42 °C | 0.1 X | 15 min. |
| C | DB33 | DED | 50 °C | 5 X | 42 °C | 0.1 X | 10 min. |
| C | DB59 | EAE | 50 °C | 5 X | 42 °C | 0.1 X | 10 min. |
| C | DB101 | DEE | 50 °C | 1 X | 42 °C | 0.1 X | 10 min. |
| D | DB34 | IK | 55 °C | 2 X | 42 °C | 0.1 X | 15 min. |
| D | DB37 | IE | 55 °C | 1 X | 42 °C | 0.1 X | 15 min. |
| D | DB38 | LK | 55 °C | 1 X | 42 °C | 0.1 X | 15 min. |
| D | DB62 | LE | 50 °C | 3 X | 42 °C | 0.1 X | 15 min. |
| D | DB63 | LR | 55 °C | 2 X | 42 °C | 0.1 X | 10 min. |
| E | AB96 | M | 42 °C | 1 X | 50 °C | 0.2 X | 15 min. |
| E | AB97 | V | 42 °C | 2 X | 50 °C | 0.2 X | 15 min. |
| E | AB98 | I | 42 °C | 2 X | 50 °C | 0.4 X | 15 min. |
| F | DB77 | DEAV | 50 °C | 3 X | 42 °C | 0.1 X | 10 min. |
| F | AB122 | GGPM | 55 °C | 3 X | 55 °C | 0.1 X | 15 min. |
| F | AB123 | VGPM | 55 °C | 3 X | 55 °C | 0.1 X | 15 min. |
| "ALL" | DB123 | "ALL" | 50 °C | 3 X | 42 °C | 0.1 X | 10 min. |

The 25-probe assay was used to characterize the allelic polymorphism in several different populations at a time when only 20 alleles were known to exist. A number of samples displayed unique hybridization patterns suggesting the existence of 10 alleles not previously observed. These putative novel alleles were cloned into m13 vectors and sequenced essentially as described in the Examples, above, using amplification primers UG21 and AB111. The primer AB111 is identical to the primer UG19, with the addition of a BamH1 site at the 5' end to facilitate cloning. The nucleotide sequences of UG19 and UG21 are provided in Example 2, above, and in the Sequence Listing section. The sequence of AB111 is shown below and in the Sequence Listing Section.

Transformants were screened for the presence of the DPB1 second exon using a PCR-based assay. Phage DNA was transferred by pipette tip into a 100 µl PCR reaction mixture containing 50 mM KCL, 10 mM Tris pH 8.3, 200 µM each dNTP, 4 mM $MgCl_2$, 2.5 Units of Taq polymerase (Perkin Elmer, Norwalk, CT), and 20 pmoles of each of the primers RS348 and RS349 (sequences shown below) which flank the m13 cloning site. Alter 35 cycles of amplification (denaturation at 95 °C for 1 minute, annealing at 60 °C for 30 seconds, extension of 72 °C for 30 seconds), 5 µl of PCR product was run on a 3% Nusieve/1% Agarose gel and clones which produced a PCR product of the correct size (approximately 400 base pairs) were sequenced using the dideoxy chain termination method with $^{35}$S-dATP and Sequenase 2.0 (United States Biochemicals).

| Primer | Seq Id. No. | Sequence |
|---|---|---|
| AB111 | SEQ ID NO: 91 | 5'GGGATCCGAGAGTGGCGCCTCCGCTCAT |
| RS348 | SEQ ID NO: 142 | 5'ACACAGGAAACAGCTATGACCATG |
| RS349 | SEQ ID NO: 143 | 5'CCAGGGTTTTCCCAGTCACGAC |

The 10 new alleles, the populations in which each of the 10 new alleles was found, and the allele frequency within that population are shown in the Table below. For each allele, two designations are given. The first is the official name assigned by the WHO Nomenclature committee; the second name shown below in parentheses is an equivalent name. The nucleotide sequences of these 10 new alleles are shown in allele sequence tables, above, and in the Sequence Listing section. The sequences have been submitted to the Genbank nucleotide sequence database and have been assigned the accession numbers M84617-M84626.

| Newly Discovered Alleles | | | |
|---|---|---|---|
| Allele | Population | Allele Frequency N/Total | Decimal Value |
| DPB1*2801 (DPB21) | SE Asians<br>Indonesians | 11/216<br>2/276 | .05<br>.007 |
| DPB1*3101 (DPB22) | SE Asians<br>Indonesians<br>Gambians | 1/216<br>12/276<br>3/1284 | .005<br>.043<br>.002 |
| DPB1*2701 (DPB23) | Hispanics<br>African Americans<br>New Guineans<br>Gambians | 2/200<br>4/292<br>1/268<br>27/1284 | .01<br>.014<br>.004<br>.021 |
| DPB1*3201 (DPB24)<br>DPB1*3301 (DPB25) | New Guineans<br>Hispanics | 1/268<br>1/200 | .004<br>.005 |
| DPB1*3401 (DPB26) | Hispanics<br>Mexican Americans | 1/200<br>1/200 | .005<br>.005 |
| DPB1*2901 (DPB27) | African Americans<br>New Guineans<br>Indonesians | 1/200<br>3/268<br>1/276 | .005<br>.011<br>.004 |
| DPB1*3001 (DPB28) | African Americans<br>Gambians<br>Sudanese | 1/162<br>5/1284<br>8/? | .006<br>.004<br>? |
| DPB1*3501 (DPB29) | Gambians<br>Portuguese | 6/1284<br>1/? | .005<br>? |
| DPB1*2101 (DPB30) | SE Asians<br>Aus Aborigines | 10/216<br>1/34 | .046<br>.029 |

Most of the alleles appear to be relatively infrequent (<5%) in the populations examined. In all but two instances, DPB1*3201 and DPB1*3301, each allele was found in a minimum of two unrelated individuals. In general, these 10 new alleles display the same patchwork pattern of polymorphism identified in the 20 DPB1 alleles described in the previous examples. DPB1*3201 is unique in that it has a single nucleotide substitution (A -> T) in the codon specifying amino acid 57 resulting in a valine residue at this position and a new sequence motif (DEV) in the third region (region C) of variability. The probe AB112 (SEQ ID NO: 92) was designed to detect this new sequence motif. The sequence of AB112 (SEQ ID No: 92) is shown below:

| | | |
|---|---|---|
| AB112 | SEQ ID NO: 92 | 5' XCCTGATGAGGTGTACTG |

where X indicates HRP. This probe has been used to type close to 2,000 individuals in a variety of populations, and on so far it has only hybridized with the original sample in which the DEV motif was found suggesting it is a rare variant.

Two of the alleles, DPB1*3101 and DPB1*3401, have a single base alteration (G -> T) at nucleotide position 214 resulting in a single amino acid change (V -> L) at position 72. This is the first example of a polymorphic position outside of the six positions herein designated A through F.

This DPB1 typing assay consists of 25 sequence specific oligonucleotide probes: 4 for Region A (LFQG, VYQL, VYQG, VHQL), 5 for Region B (EEFARF, EELVRF, QEYARF, EEYARF, and EEFVRF) 4 for Region C (AAE, DEE, EAE, DED), 5 for Region D (I-K, I-E, L-K, L-E, L-R), 3 for Region E (M, V, I), and 2 for Region F (GGPM,VGPM, DEAV). With the original 20 DPB1 alleles that were known before the discovery of the 10 new alleles discussed above, this assay was able to distinguish all but 6 of the 190 heterozygous genotypes (with 20 alleles there are 210 possible genotypes, 190 of which are heterozygous). The addition of the 10 new alleles reported here increases the number of DPB1 alleles to 30, and the number of possible genotypes to 465. The addition of these new alleles introduces additional ambiguities into the assay such that certain heterozygous combinations may have indistinguishable probe hybridization patterns. However, with the use of the 25 probes typing assay plus the probe for the DEV motif (AB112 - SEQ ID No: 92) all but 9 pairs of genotypes (18 genotypes) are distingnishable.

With the study of more populations, it is expected that new alleles will be discovered. The methods of the present invention provide means to detect new alleles and are not limited to the alleles described above. Rapid and accurate DPB1 typing is accomplished by the selection of suitable probe panels by the methods described herein.

SEQUENCE LISTING

(1) GENERAL INFORMATION:

(i) APPLICANT:
(A) NAME: F.HOFFMANN-LA ROCHE AG
(B) STREET: Grenzacherstrasse 124
(C) CITY: Basel
(D) STATE: BS
(E) COUNTRY: Switzerland
(F) POSTAL CODE (ZIP): CH-4002
(G) TELEPHONE: (0)61 - 688 24 03
(H) TELEFAX: (0)61 - 688 13 95
(I) TELEX: 962292/965542 hlr ch

(ii) TITLE OF INVENTION: Method for HLA-DP typing

(iii) NUMBER OF SEQUENCES: 157

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(vi) PRIOR APPLICATION DATA:
(A) APPLICATION NUMBER: US 07/903,028
(B) FILING DATE: 23-JUN-1992

(2) INFORMATION FOR SEQ ID NO:1:
(i) SEQUENCE CHARACTERISTICS:
(A) LENGTH: 81 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:1:

```
Asp His Val Ser Thr Tyr Ala Ala Phe Val Gln Thr His Arg Pro Thr
1               5                   10                  15

Gly Glu Phe Met Phe Glu Phe Asp Glu Asp Glu Met Phe Tyr Val Asp
            20                  25                  30

Leu Asp Lys Lys Glu Thr Val Trp His Leu Glu Glu Phe Gly Gln Ala
        35                  40                  45

Phe Ser Phe Glu Ala Gln Gly Gly Leu Ala Asn Ile Ala Ile Leu Asn
    50                  55                  60

Asn Asn Leu Asn Thr Leu Ile Gln Arg Ser Asn His Thr Gln Ala Thr
65                  70                  75                  80

Asn
```

(2) INFORMATION FOR SEQ ID NO:2:
(i) SEQUENCE CHARACTERISTICS:
(A) LENGTH: 81 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:2:

```
Asp His Val Ser Thr Tyr Ala Ala Phe Val Gln Thr His Arg Pro Thr
1               5                   10                  15

Gly Glu Phe Met Phe Glu Phe Asp Glu Asp Glu Gln Phe Tyr Val Asp
            20                  25                  30

Leu Asp Lys Lys Glu Thr Val Trp His Leu Glu Glu Phe Gly Arg Ala
        35                  40                  45

Phe Ser Phe Glu Ala Gln Gly Gly Leu Ala Asn Ile Ala Ile Leu Asn
    50                  55                  60

Asn Asn Leu Asn Thr Leu Ile Gln Arg Ser Asn His Thr Gln Ala Ala
65                  70                  75                  80

Asn
```

(2) INFORMATION FOR SEQ ID NO:3:
(i) SEQUENCE CHARACTERISTICS:
(A) LENGTH: 80 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:3:

```
Asp His Val Ser Thr Tyr Ala Glu Phe Val Gln Thr His Arg Pro Ser
1               5                   10                  15

Gly Glu Tyr Met Phe Glu Phe Asp Glu Glu Glu Gln Phe Tyr Val Asn
            20                  25                  30

Leu Asp Glu Lys Glu Met Val Trp Pro Leu Pro Glu Phe Ile His Thr
        35                  40                  45

Phe Asp Phe Gly Ala Gln Arg Gly Ile Ala Gly Ile Val Met Ala Arg
    50                  55                  60

Lys His Leu Asn Thr Arg Ile Asn Gly Lys Gln Thr Trp Ala Thr Asp
65                  70                  75                  80
```

(2) INFORMATION FOR SEQ ID NO:4:
(i) SEQUENCE CHARACTERISTICS:
(A) LENGTH: 87 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:4:

```
Asn Ser Val Tyr Gln Glu Arg Gln Glu Cys Tyr Ala Phe Asn Gly Thr
1               5                   10                  15

Gln Arg Val Val Asp Gly Leu Ile Tyr Asn Arg Glu Glu Tyr Val His
            20                  25                  30

Phe Asp Ala Asp Val Gly Glu Leu Arg Ala Met Thr Glu Leu Gly Arg
        35                  40                  45

Pro Ile Gly Glu Tyr Phe Asn Ser Gln Lys Asp Phe Met Glu Arg Lys
    50                  55                  60

Arg Ala Glu Val Asp Lys Val Cys Arg His Lys Tyr Glu Leu Met Glu
65                  70                  75                  80

Pro Leu Ile Arg Gln Arg Arg
                85
```

(2) INFORMATION FOR SEQ ID NO:5:
(i) SEQUENCE CHARACTERISTICS:
(A) LENGTH: 249 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:5:

```
GTGTACCAGG GACGGCAGGA ATGCTACGCG TTTAATGGGA CACAGCGCTT CCTGGAGAGA    60

TACATCTACA ACCGGGAGGA GTACGCGCGC TTCGACAGCG ACGTGGGGGA GTTCCGGGCG   120

GTGACGGAGC TGGGGCGGCC TGCTGCGGAG TACTGGAACA GCCAGAAGGA CATCCTGGAG   180

GAGAAGCGGG CAGTGCCGGA CAGGGTATGC AGACACAACT ACGAGCTGGA CGAGGCCGTG   240

ACCCTGCAG                                                          249
```

(2) INFORMATION FOR SEQ ID NO:6:
(i) SEQUENCE CHARACTERISTICS:
(A) LENGTH: 83 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:6:

```
Val Tyr Gln Gly Arg Gln Glu Cys Tyr Ala Phe Asn Gly Thr Gln Arg
1               5                   10                  15

Phe Leu Glu Arg Tyr Ile Tyr Asn Arg Glu Glu Tyr Ala Arg Phe Asp
            20                  25                  30

Ser Asp Val Gly Glu Phe Arg Ala Val Thr Glu Leu Gly Arg Pro Ala
        35                  40                  45

Ala Glu Tyr Trp Asn Ser Gln Lys Asp Ile Leu Glu Glu Lys Arg Ala
    50                  55                  60

Val Pro Asp Arg Val Cys Arg His Asn Tyr Glu Leu Asp Glu Ala Val
65                  70                  75                      80

Thr Leu Gln
```

(2) INFORMATION FOR SEQ ID NO:7:
(i) SEQUENCE CHARACTERISTICS:
(A) LENGTH: 257 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:7:

```
AGAATTACCT TTTCCAGGGA CGGCAGGAAT GCTACGCGTT TAATGGGACA CAGCGCTTCC    60

TGGAGAGATA CATCTACAAC CGGGAGGAGT TCGTGCGCTT CGACAGCGAC GTGGGGGAGT   120

TCCGGGCGGT GACGGAGCTG GGGCGGCCTG ATGAGGAGTA CTGGAACAGC CAGAAGGACA   180

TCCTGGAGGA GGAGCGGGCA GTGCCGGACA GGATGTGCAG ACACAACTAC GAGCTGGGCG   240

GGCCCATGAC CCTGCAG                                                  257
```

(2) INFORMATION FOR SEQ ID NO:8:
(i) SEQUENCE CHARACTERISTICS:
(A) LENGTH: 87 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:8:

```
Asn Tyr Leu Phe Gln Gly Arg Gln Glu Cys Tyr Ala Phe Asn Gly Thr
1               5                   10                  15

Gln Arg Phe Leu Glu Arg Tyr Ile Tyr Asn Arg Glu Glu Phe Val Arg
            20                  25                  30

Phe Asp Ser Asp Val Gly Glu Phe Arg Ala Val Thr Glu Leu Gly Arg
        35                  40                  45

Pro Asp Glu Glu Tyr Trp Asn Ser Gln Lys Asp Ile Leu Glu Glu Glu
```

```
        50                  55                  60

    Arg Ala Val Pro Asp Arg Met Cys Arg His Asn Tyr Glu Leu Gly Gly
    65                  70                  75                  80

    Pro Met Thr Leu Gln Arg Arg
                    85
```

(2) INFORMATION FOR SEQ ID NO:9:
(i) SEQUENCE CHARACTERISTICS:
(A) LENGTH: 249 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:9:

```
CTTTTCCAGG GACGGCAGGA ATGCTACGCG TTTAATGGGA CACAGCGCTT CCTGGAGAGA     60

TACATCTACA ACCGGGAGGA GCTCGTGCGC TTCGACAGCG ACGTGGGGGA GTTCCGGGCG    120

GTGACGGAGC TGGGGCGGCC TGAGGCGGAG TACTGGAACA GCCAGAAGGA CATCCTGGAG    180

GAGGAGCGGG CAGTGCCGGA CAGGATGTGC AGACACAACT ACGAGCTGGG CGGGCCCATG    240

ACCCTGCAG                                                            249
```

(2) INFORMATION FOR SEQ ID NO:10:
(i) SEQUENCE CHARACTERISTICS:
(A) LENGTH: 85 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:10:

```
    Asn Tyr Leu Phe Gln Gly Arg Gln Glu Cys Tyr Ala Phe Asn Gly Thr
    1               5                   10                  15

    Gln Arg Phe Leu Glu Arg Tyr Ile Tyr Asn Arg Glu Glu Phe Ala Arg
                20                  25                  30

    Phe Asp Ser Asp Val Gly Glu Phe Arg Ala Val Thr Glu Leu Gly Arg
            35                  40                  45

    Pro Ala Ala Glu Tyr Trp Asn Ser Gln Lys Asp Leu Leu Glu Glu Lys
        50                  55                  60

    Arg Ala Leu Pro Asp Arg Met Cys Arg His Asn Tyr Glu Leu Asp Glu
    65                  70                  75                  80

    Ala Val Thr Leu Gln
                    85
```

(2) INFORMATION FOR SEQ ID NO:11:
(i) SEQUENCE CHARACTERISTICS:

(A) LENGTH: 249 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:11:

```
GTGTACCAGT TACGGCAGGA ATGCTACGCG TTTAATGGGA CACAGCGCTT CCTGGAGAGA      60

TACATCTACA ACCGGGAGGA GTTCGTGCGC TTCGACAGCG ACGTGGGGGA GTTCCGGGCG     120

GTGACGGAGC TGGGGCGGCC TGATGAGGAC TACTGGAACA GCCAGAAGGA CCTCCTGGAG     180

GAGAAGCGGG CAGTGCCGGA CAGGGTATGC AGACACAACT ACGAGCTGGA CGAGGCCGTG     240

ACCCTGCAG                                                             249
```

(2) INFORMATION FOR SEQ ID NO:12:
(i) SEQUENCE CHARACTERISTICS:
(A) LENGTH: 83 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:12:

```
Val Tyr Gln Leu Arg Gln Glu Cys Tyr Ala Phe Asn Gly Thr Gln Arg
1               5                   10                  15

Phe Leu Glu Arg Tyr Ile Tyr Asn Arg Glu Glu Phe Val Arg Phe Asp
            20                  25                  30

Ser Asp Val Gly Glu Phe Arg Ala Val Thr Glu Leu Gly Arg Pro Asp
        35                  40                  45

Glu Asp Tyr Trp Asn Ser Gln Lys Asp Leu Leu Glu Glu Lys Arg Ala
    50                  55                  60

Val Pro Asp Arg Val Cys Arg His Asn Tyr Glu Leu Asp Glu Ala Val
65                  70                  75                  80

Thr Leu Gln
```

(2) INFORMATION FOR SEQ ID NO:13:
(i) SEQUENCE CHARACTERISTICS:
(A) LENGTH: 264 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:13:

```
AGAATTACCT TTTCCAGGGA CGGCAGGAAT GCTACGCGTT TAATGGGACA CAGCGCTTCC      60

TGGAGAGATA CATCTACAAC CGGGAGGAGT TCGCGCGCTT CGACAGCGAC GTGGGGGAGT     120
```

TCCGGGCGGT GACGGAGCTG GGGCGGCCTG CTGCGGAGTA CTGGAACAGC CAGAAGGACA 180

TCCTGGAGGA GAAGCGGGCA GTGCCGGACA GGATGTGCAG ACACAACTAC GAGCTGGGCG 240

GGCCCATGAC CCTGCAGCGC CGAG 264

(2) INFORMATION FOR SEQ ID NO:14:
(i) SEQUENCE CHARACTERISTICS:
(A) LENGTH: 87 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:14:

```
Asn Tyr Leu Phe Gln Gly Arg Gln Glu Cys Tyr Ala Phe Asn Gly Thr
1               5                   10                  15

Gln Arg Phe Leu Glu Arg Tyr Ile Tyr Asn Arg Glu Glu Phe Ala Arg
            20                  25                  30

Phe Asp Ser Asp Val Gly Glu Phe Arg Ala Val Thr Glu Leu Gly Arg
        35                  40                  45

Pro Ala Ala Glu Tyr Trp Asn Ser Gln Lys Asp Ile Leu Glu Glu Lys
    50                  55                  60

Arg Ala Val Pro Asp Arg Met Cys Arg His Asn Tyr Glu Leu Gly Gly
65                  70                  75                  80

Pro Met Thr Leu Gln Arg Arg
                85
```

(2) INFORMATION FOR SEQ ID NO:15:
(i) SEQUENCE CHARACTERISTICS:
(A) LENGTH: 256 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:15:

CTTTTCCAGG GACGGCAGGA ATGCTACGCG TTTAATGGGA CACAGCGCTT CCTGGAGAGA 60

TACATCTACA ACCGGGAGGA GTTCGTGCGC TTCGACAGCG ACGTGGGGGA GTTCCGGGCG 120

GTGACGGAGC TGGGGCGGCC TGATGAGGAG TACTGGAACA GCCAGAAGGA CATCCTGGAG 180

GAGAAGCGGG CAGTGCCGGA CAGGATGTGC AGACACAACT ACGAGCTGGG CGGGCCCATG 240

ACCCTGCAGC GCCGAG 256

(2) INFORMATION FOR SEQ ID NO:16:
(i) SEQUENCE CHARACTERISTICS:
(A) LENGTH: 83 amino acids

(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:16:

```
Leu Phe Gln Gly Arg Gln Glu Cys Tyr Ala Phe Asn Gly Thr Gln Arg
1               5                   10                  15

Phe Leu Glu Arg Tyr Ile Tyr Asn Arg Glu Glu Phe Val Arg Phe Asp
            20                  25                  30

Ser Asp Val Gly Glu Phe Arg Ala Val Thr Glu Leu Gly Arg Pro Asp
        35                  40                  45

Glu Glu Tyr Trp Asn Ser Gln Lys Asp Ile Leu Glu Glu Lys Arg Ala
    50                  55                  60

Val Pro Asp Arg Met Cys Arg His Asn Tyr Glu Leu Gly Gly Pro Met
65                  70                  75                  80

Thr Leu Gln
```

(2) INFORMATION FOR SEQ ID NO:17:
(i) SEQUENCE CHARACTERISTICS:
(A) LENGTH: 249 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:17:

```
CTTTTCCAGG GACGGCAGGA ATGCTACGCG TTTAATGGGA CACAGCGCTT CCTGGAGAGA    60

TACATCTACA ACCGGGAGGA GCTCGTGCGC TTCGACAGCG ACGTGGGGGA GTTCCGGGCG   120

GTGACGGAGC TGGGGCGGCC TGAGGCGGAG TACTGGAACA GCCAGAAGGA CATCCTGGAG   180

GAGAAGCGGG CAGTGCCGGA CAGGATGTGC AGACACAACT ACGAGCTGGA CGAGGCCGTG   240

ACCCTGCAG                                                           249
```

(2) INFORMATION FOR SEQ ID NO:18:
(i) SEQUENCE CHARACTERISTICS:
(A) LENGTH: 83 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:18:

```
Leu Phe Gln Gly Arg Gln Glu Cys Tyr Ala Phe Asn Gly Thr Gln Arg
1               5                   10                  15
```

```
Phe Leu Glu Arg Tyr Ile Tyr Asn Arg Glu Glu Leu Val Arg Phe Asp
            20                  25                  30

Ser Asp Val Gly Glu Phe Arg Ala Val Thr Glu Leu Gly Arg Pro Glu
        35                  40                  45

Ala Glu Tyr Trp Asn Ser Gln Lys Asp Ile Leu Glu Glu Lys Arg Ala
    50                  55                  60

Val Pro Asp Arg Met Cys Arg His Asn Tyr Glu Leu Asp Glu Ala Val
65                  70                  75                  80

Thr Leu Gln
```

(2) INFORMATION FOR SEQ ID NO:19:
(i) SEQUENCE CHARACTERISTICS:
(A) LENGTH: 249 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:19:

```
GTGTACCAGT TACGGCAGGA ATGCTACGCG TTTAATGGGA CACAGCGCTT CCTGGAGAGA    60

TACATCTACA ACCGGGAGGA GTTCGTGCGC TTCGACAGCG ACGTGGGGGA GTTCCGGGCG   120

GTGACGGAGC TGGGGCGGCC TGATGAGGAC TACTGGAACA GCCAGAAGGA CCTCCTGGAG   180

GAGGAGCGGG CAGTGCCGGA CAGGATGTGC AGACACAACT ACGAGCTGGA CGAGGCCGTG   240

ACCCTGCAG                                                           249
```

(2) INFORMATION FOR SEQ ID NO:20:
(i) SEQUENCE CHARACTERISTICS:
(A) LENGTH: 83 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:20:

```
Val Tyr Gln Leu Arg Gln Glu Cys Tyr Ala Phe Asn Gly Thr Gln Arg
1               5                   10                  15

Phe Leu Glu Arg Tyr Ile Tyr Asn Arg Glu Glu Phe Val Arg Phe Asp
            20                  25                  30

Ser Asp Val Gly Glu Phe Arg Ala Val Thr Glu Leu Gly Arg Pro Asp
        35                  40                  45

Glu Asp Tyr Trp Asn Ser Gln Lys Asp Leu Leu Glu Glu Glu Arg Ala
    50                  55                  60

Val Pro Asp Arg Met Cys Arg His Asn Tyr Glu Leu Asp Glu Ala Val
65                  70                  75                  80
```

Thr Leu Gln

(2) INFORMATION FOR SEQ ID NO:21:
(i) SEQUENCE CHARACTERISTICS:
(A) LENGTH: 249 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:21:

CTTTTCCAGG GACGGCAGGA ATGCTACGCG TTTAATGGGA CACAGCGCTT CCTGGAGAGA 60

TACATCTACA ACCGGGAGGA GTTCGTGCGC TTCGACAGCG ACGTGGGGGA GTTCCGGGCG 120

GTGACGGAGC TGGGGCGGCC TGATGAGGAG TACTGGAACA GCCAGAAGGA CATCCTGGAG 180

GAGGAGCGGG CAGTGCCGGA CAGGGTATGC AGACACAACT ACGAGCTGGA CGAGGCCGTG 240

ACCCTGCAG 249

(2) INFORMATION FOR SEQ ID NO:22:
(i) SEQUENCE CHARACTERISTICS:
(A) LENGTH: 83 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:22:

Leu Phe Gln Gly Arg Gln Glu Cys Tyr Ala Phe Asn Gly Thr Gln Arg
1 5 10 15

Phe Leu Glu Arg Tyr Ile Tyr Asn Arg Glu Glu Phe Val Arg Phe Asp
20 25 30

Ser Asp Val Gly Glu Phe Arg Ala Val Thr Glu Leu Gly Arg Pro Asp
35 40 45

Glu Glu Tyr Trp Asn Ser Gln Lys Asp Ile Leu Glu Glu Glu Arg Ala
50 55 60

Val Pro Asp Arg Val Cys Arg His Asn Tyr Glu Leu Asp Glu Ala Val
65 70 75 80

Thr Leu Gln

(2) INFORMATION FOR SEQ ID NO:23:
(i) SEQUENCE CHARACTERISTICS:
(A) LENGTH: 249 base pairs
(B) TYPE: nucleic acid
(C) STRANDEDNESS: single
(D) TOPOLOGY: linear
(ii) MOLECULE TYPE: DNA (genomic)

(xi) SEQUENCE DESCRIPTION: SEQ ID NO:23:

```
GTGCACCAGT TACGGCAGGA ATGCTACGCG TTTAATGGGA CACAGCGCTT CCTGGAGAGA    60
TACATCTACA ACCGGGAGGA GTTCGTGCGC TTCGACAGCG ACGTGGGGGA GTTCCGGGCG   120
GTGACGGAGC TGGGGCGGCC TGATGAGGAC TACTGGAACA GCCAGAAGGA CATCCTGGAG   180
GAGGAGCGGG CAGTGCCGGA CAGGGTATGC AGACACAACT ACGAGCTGGA CGAGGCCGTG   240
ACCCTGCAG                                                           249
```

(2) INFORMATION FOR SEQ ID NO:24:

(i) SEQUENCE CHARACTERISTICS:

(A) LENGTH: 83 amino acids

(B) TYPE: amino acid

(C) STRANDEDNESS: single

(D) TOPOLOGY: linear

(ii) MOLECULE TYPE: peptide

(xi) SEQUENCE DESCRIPTION: SEQ ID NO:24:

```
Val His Gln Leu Arg Gln Glu Cys Tyr Ala Phe Asn Gly Thr Gln Arg
1               5                   10                  15

Phe Leu Glu Arg Tyr Ile Tyr Asn Arg Glu Glu Phe Val Arg Phe Asp
            20                  25                  30

Ser Asp Val Gly Glu Phe Arg Ala Val Thr Glu Leu Gly Arg Pro Asp
        35                  40                  45

Glu Asp Tyr Trp Asn Ser Gln Lys Asp Ile Leu Glu Glu Glu Arg Ala
    50                  55                  60

Val Pro Asp Arg Val Cys Arg His Asn Tyr Glu Leu Asp Glu Ala Val
65                  70                  75                  80

Thr Leu Gln
```

(2) INFORMATION FOR SEQ ID NO:25:

(i) SEQUENCE CHARACTERISTICS:

(A) LENGTH: 249 base pairs

(B) TYPE: nucleic acid

(C) STRANDEDNESS: single

(D) TOPOLOGY: linear

(ii) MOLECULE TYPE: DNA (genomic)

(xi) SEQUENCE DESCRIPTION: SEQ ID NO:25:

```
GTGCACCAGT TACGGCAGGA ATGCTACGCG TTTAATGGGA CACAGCGCTT CCTGGAGAGA    60
TACATCTACA ACCGGGAGGA GTTCGTGCGC TTCGACAGCG ACGTGGGGGA GTTCCGGGCG   120
GTGACGGAGC TGGGGCGGCC TGATGAGGAG TACTGGAACA GCCAGAAGGA CATCCTGGAG   180
GAGGAGCGGG CAGTGCCGGA CAGGGTATGC AGACACAACT ACGAGCTGGA CGAGGCCGTG   240
ACCCTGCAG                                                           249
```

(2) INFORMATION FOR SEQ ID NO:26:
(i) SEQUENCE CHARACTERISTICS:
(A) LENGTH: 83 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:26:

```
Val His Gln Leu Arg Gln Glu Cys Tyr Ala Phe Asn Gly Thr Gln Arg
1               5                   10                  15

Phe Leu Glu Arg Tyr Ile Tyr Asn Arg Glu Glu Phe Val Arg Phe Asp
            20                  25                  30

Ser Asp Val Gly Glu Phe Arg Ala Val Thr Glu Leu Gly Arg Pro Asp
        35                  40                  45

Glu Glu Tyr Trp Asn Ser Gln Lys Asp Ile Leu Glu Glu Glu Arg Ala
    50                  55                  60

Val Pro Asp Arg Val Cys Arg His Asn Tyr Glu Leu Asp Glu Ala Val
65                  70                  75                  80

Thr Leu Gln
```

(2) INFORMATION FOR SEQ ID NO:27:
(i) SEQUENCE CHARACTERISTICS:
(A) LENGTH: 249 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:27:

```
GTGTACCAGT TACGGCAGGA ATGCTACGCG TTTAATGGGA CACAGCGCTT CCTGGAGAGA    60

TACATCTACA ACCGGCAGGA GTACGCGCGC TTCGACAGCG ACGTGGGAGA GTTCCGGGCG   120

GTGACGGAGC TGGGGCGGCC TGCTGCGGAG TACTGGAACA GCCAGAAGGA CCTCCTGGAG   180

GAGAGGCGGG CAGTGCCGGA CAGGATGTGC AGACACAACT ACGAGCTGGA CGAGGCCGTG   240

ACCCTGCAG                                                           249
```

(2) INFORMATION FOR SEQ ID NO:28:
(i) SEQUENCE CHARACTERISTICS:
(A) LENGTH: 83 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:28:

Val Tyr Gln Leu Arg Gln Glu Cys Tyr Ala Phe Asn Gly Thr Gln Arg
1 5 10 15

Phe Leu Glu Arg Tyr Ile Tyr Asn Arg Gln Glu Tyr Ala Arg Phe Asp
20 25 30

Ser Asp Val Gly Glu Phe Arg Ala Val Thr Glu Leu Gly Arg Pro Ala
35 40 45

Ala Glu Tyr Trp Asn Ser Gln Lys Asp Leu Leu Glu Glu Arg Arg Ala
50 55 60

Val Pro Asp Arg Met Cys Arg His Asn Tyr Glu Leu Asp Glu Ala Val
65 70 75 80

Thr Leu Gln

(2) INFORMATION FOR SEQ ID NO:29:
(i) SEQUENCE CHARACTERISTICS:
(A) LENGTH: 249 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:29:

GTGTACCAGT TACGGCAGGA ATGCTACGCG TTTAATGGGA CACAGCGCTT CCTGGAGAGA 60

TACATCTACA ACCGGGAGGA GTACGCGCGC TTCGACAGCG ACGTGGGAGA GTTCCGGGCG 120

GTGACGGAGC TGGGGCGGCC TGCTGCGGAG TACTGGAACA GCCAGAAGGA CATCCTGGAG 180

GAGGAGCGGG CAGTGCCGGA CAGGATATGC AGACACAACT ACGAGCTGGA CGAGGCCGTG 240

ACCCTGCAG 249

(2) INFORMATION FOR SEQ ID NO:30:
(i) SEQUENCE CHARACTERISTICS:
(A) LENGTH: 83 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:30:

Val Tyr Gln Leu Arg Gln Glu Cys Tyr Ala Phe Asn Gly Thr Gln Arg
1 5 10 15

Phe Leu Glu Arg Tyr Ile Tyr Asn Arg Glu Glu Tyr Ala Arg Phe Asp
20 25 30

Ser Asp Val Gly Glu Phe Arg Ala Val Thr Glu Leu Gly Arg Pro Ala
35 40 45

Ala Glu Tyr Trp Asn Ser Gln Lys Asp Ile Leu Glu Glu Glu Arg Ala

```
            50                  55                  60

        Val Pro Asp Arg Ile Cys Arg His Asn Tyr Glu Leu Asp Glu Ala Val
        65                  70                  75                  80

        Thr Leu Gln
```

(2) INFORMATION FOR SEQ ID NO:31:
(i) SEQUENCE CHARACTERISTICS:
(A) LENGTH: 249 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:31:

```
GTGCACCAGT TACGGCAGGA ATGCTACGCG TTTAATGGGA CACAGCGCTT CCTGGAGAGA    60

TACATCTACA ACCGGGAGGA GTTCGTGCGC TTCGACAGCG ACGTGGGGGA GTTCCGGGCG   120

GTGACGGAGC TGGGGCGGCC TGATGAGGAC TACTGGAACA GCCAGAAGGA CCTCCTGGAG   180

GAGAAGCGGG CAGTGCCGGA CAGGGTATGC AGACACAACT ACGAGCTGGA CGAGGCCGTG   240

ACCCTGCAG                                                           249
```

(2) INFORMATION FOR SEQ ID NO:32:
(i) SEQUENCE CHARACTERISTICS:
(A) LENGTH: 83 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:32:

```
Val His Gln Leu Arg Gln Glu Cys Tyr Ala Phe Asn Gly Thr Gln Arg
1               5                   10                  15

Phe Leu Glu Arg Tyr Ile Tyr Asn Arg Glu Glu Phe Val Arg Phe Asp
            20                  25                  30

Ser Asp Val Gly Glu Phe Arg Ala Val Thr Glu Leu Gly Arg Pro Asp
        35                  40                  45

Glu Asp Tyr Trp Asn Ser Gln Lys Asp Leu Leu Glu Glu Lys Arg Ala
    50                  55                  60

Val Pro Asp Arg Val Cys Arg His Asn Tyr Glu Leu Asp Glu Ala Val
65                  70                  75                  80

Thr Leu Gln
```

(2) INFORMATION FOR SEQ ID NO:33:
(i) SEQUENCE CHARACTERISTICS:

(A) LENGTH: 249 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:33:

```
GTGTACCAGG GACGGCAGGA ATGCTACGCG TTTAATGGGA CACAGCGCTT CCTGGAGAGA    60
TACATCTACA ACCGGCAGGA GTACGCGCGC TTCGACAGCG ACGTGGGAGA GTTCCGGGCG   120
GTGACGGAGC TGGGGCGGCC TGCTGCGGAG TACTGGAACA GCCAGAAGGA CCTCCTGGAG   180
GAGAGGCGGG CAGTGCCGGA CAGGATGTGC AGACACAACT ACGAGCTGGT CGGGCCCATG   240
ACCCTGCAG                                                           249
```

(2) INFORMATION FOR SEQ ID NO:34:
(i) SEQUENCE CHARACTERISTICS:
(A) LENGTH: 83 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:34:

```
Val Tyr Gln Gly Arg Gln Glu Cys Tyr Ala Phe Asn Gly Thr Gln Arg
1               5                   10                  15

Phe Leu Glu Arg Tyr Ile Tyr Asn Arg Gln Glu Tyr Ala Arg Phe Asp
            20                  25                  30

Ser Asp Val Gly Glu Phe Arg Ala Val Thr Glu Leu Gly Arg Pro Ala
        35                  40                  45

Ala Glu Tyr Trp Asn Ser Gln Lys Asp Leu Leu Glu Glu Arg Arg Ala
    50                  55                  60

Val Pro Asp Arg Met Cys Arg His Asn Tyr Glu Leu Val Gly Pro Met
65                  70                  75                  80

Thr Leu Gln
```

(2) INFORMATION FOR SEQ ID NO:35:
(i) SEQUENCE CHARACTERISTICS:
(A) LENGTH: 249 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:35:

```
CTTTTCCAGG GACGGCAGGA ATGCTACGCG TTTAATGGGA CACAGCGCTT CCTGGAGAGA    60
TACATCTACA ACCGGGAGGA GTTCGTGCGC TTCGACAGCG ACGTGGGGGA GTTCCGGGCG   120
```

GTGACGGAGC TGGGGCGGCC TGATGAGGAG TACTGGAACA GCCAGAAGGA CATCCTGGAG 180

GAGGAGCGGG CAGTGCCGGA CAGGATGTGC AGACACAACT ACGAGCTGGA CGAGGCCGTG 240

ACCCTGCAG 249

(2) INFORMATION FOR SEQ ID NO:36:
(i) SEQUENCE CHARACTERISTICS:
(A) LENGTH: 83 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:36:

```
Leu Phe Gln Gly Arg Gln Glu Cys Tyr Ala Phe Asn Gly Thr Gln Arg
1               5                   10                  15

Phe Leu Glu Arg Tyr Ile Tyr Asn Arg Glu Glu Phe Val Arg Phe Asp
            20                  25                  30

Ser Asp Val Gly Glu Phe Arg Ala Val Thr Glu Leu Gly Arg Pro Asp
        35                  40                  45

Glu Glu Tyr Trp Asn Ser Gln Lys Asp Ile Leu Glu Glu Glu Arg Ala
    50                  55                  60

Val Pro Asp Arg Met Cys Arg His Asn Tyr Glu Leu Asp Glu Ala Val
65                  70                  75                  80

Thr Leu Gln
```

(2) INFORMATION FOR SEQ ID NO:37:
(i) SEQUENCE CHARACTERISTICS:
(A) LENGTH: 249 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:37:

GTGCACCAGT TACGGCAGGA ATGCTACGCG TTTAATGGGA CACAGCGCTT CCTGGAGAGA 60

TACATCTACA ACCGGGAGGA GTTCGTGCGC TTCGACAGCG ACGTGGGGGA GTTCCGGGCG 120

GTGACGGAGC TGGGGCGGCC TGATGAGGAC TACTGGAACA GCCAGAAGGA CATCCTGGAG 180

GAGGAGCGGG CAGTGCCGGA CAGGATGTGC AGACACAACT ACGAGCTGGA CGAGGCCGTG 240

ACCCTGCAG 249

(2) INFORMATION FOR SEQ ID NO:38:
(i) SEQUENCE CHARACTERISTICS:
(A) LENGTH: 83 amino acids

(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:38:

```
Val His Gln Leu Arg Gln Glu Cys Tyr Ala Phe Asn Gly Thr Gln Arg
1               5                   10                  15

Phe Leu Glu Arg Tyr Ile Tyr Asn Arg Glu Glu Phe Val Arg Phe Asp
            20                  25                  30

Ser Asp Val Gly Glu Phe Arg Ala Val Thr Glu Leu Gly Arg Pro Asp
        35                  40                  45

Glu Asp Tyr Trp Asn Ser Gln Lys Asp Ile Leu Glu Glu Glu Arg Ala
    50                  55                  60

Val Pro Asp Arg Met Cys Arg His Asn Tyr Glu Leu Asp Glu Ala Val
65                  70                  75                  80

Thr Leu Gln
```

(2) INFORMATION FOR SEQ ID NO:39:
(i) SEQUENCE CHARACTERISTICS:
(A) LENGTH: 249 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:39:

```
GTGTACCAGG GACGGCAGGA ATGCTACGCG TTTAATGGGA CACAGCGCTT CCTGGAGAGA    60

TACATCTACA ACCGGGAGGA GTTCGTGCGC TTCGACAGCG ACGTGGGGGA GTTCCGGGCG   120

GTGACGGAGC TGGGGCGGCC TGATGAGGAG TACTGGAACA GCCAGAAGGA CATCCTGGAG   180

GAGAAGCGGG CAGTGCCGGA CAGGATGTGC AGACACAACT ACGAGCTGGT CGGGCCCATG   240

ACCCTGCAG                                                           249
```

(2) INFORMATION FOR SEQ ID NO:40:
(i) SEQUENCE CHARACTERISTICS:
(A) LENGTH: 83 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:40:

```
Val Tyr Gln Gly Arg Gln Glu Cys Tyr Ala Phe Asn Gly Thr Gln Arg
1               5                   10                  15
```

Phe Leu Glu Arg Tyr Ile Tyr Asn Arg Glu Glu Phe Val Arg Phe Asp
20 25 30

Ser Asp Val Gly Glu Phe Arg Ala Val Thr Glu Leu Gly Arg Pro Asp
35 40 45

Glu Glu Tyr Trp Asn Ser Gln Lys Asp Ile Leu Glu Glu Lys Arg Ala
50 55 60

Val Pro Asp Arg Met Cys Arg His Asn Tyr Glu Leu Val Gly Pro Met
65 70 75 80

Thr Leu Gln

(2) INFORMATION FOR SEQ ID NO:41:
(i) SEQUENCE CHARACTERISTICS:
(A) LENGTH: 249 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:41:

CTTTTCCAGG GACGGCAGGA ATGCTACGCG TTTAATGGGA CACAGCGCTT CCTGGAGAGA 60

TACATCTACA ACCGGGAGGA GTTCGTGCGC TTCGACAGCG ACGTGGGGGA GTTCCGGGCG 120

GTGACGGAGC TGGGGCGGCC TGAGGCGGAG TACTGGAACA GCCAGAAGGA CATCCTGGAG 180

GAGGAGCGGG CAGTGCCGGA CAGGATATGC AGACACAACT ACGAGCTGGA CGAGGCCGTG 240

ACCCTGCAG 249

(2) INFORMATION FOR SEQ ID NO:42:
(i) SEQUENCE CHARACTERISTICS:
(A) LENGTH: 83 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:42:

Leu Phe Gln Gly Arg Gln Glu Cys Tyr Ala Phe Asn Gly Thr Gln Arg
1 5 10 15

Phe Leu Glu Arg Tyr Ile Tyr Asn Arg Glu Glu Phe Val Arg Phe Asp
20 25 30

Ser Asp Val Gly Glu Phe Arg Ala Val Thr Glu Leu Gly Arg Pro Glu
35 40 45

Ala Glu Tyr Trp Asn Ser Gln Lys Asp Ile Leu Glu Glu Glu Arg Ala
50 55 60

Val Pro Asp Arg Ile Cys Arg His Asn Tyr Glu Leu Asp Glu Ala Val
65 70 75 80

Thr Leu Gln

(2) INFORMATION FOR SEQ ID NO:43:
(i) SEQUENCE CHARACTERISTICS:
(A) LENGTH: 83 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:43:

Val His Gln Leu Arg Gln Glu Cys Tyr Ala Phe Asn Gly Thr Gln Arg
1 5 10 15

Phe Leu Glu Arg Tyr Ile Tyr Asn Arg Glu Glu Phe Val Arg Phe Asp
20 25 30

Ser Asp Val Gly Glu Phe Arg Ala Val Thr Glu Leu Gly Arg Pro Asp
35 40 45

Glu Asp Tyr Trp Asn Ser Gln Lys Asp Leu Leu Glu Glu Lys Arg Ala
50 55 60

Val Pro Asp Arg Met Cys Arg His Asn Tyr Glu Leu Asp Glu Ala Val
65 70 75 80

Thr Leu Gln

(2) INFORMATION FOR SEQ ID NO:44:
(i) SEQUENCE CHARACTERISTICS:
(A) LENGTH: 257 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:44:

AGAATTACCT TTTCCAGGGA CGGCAGGAAT GCTACGCGTT TAATGGGACA CAGCGCTTCC 60

TGGAGAGATA CATCTACAAC CGGGAGGAGT TCGCGCGCTT CGACAGCGAC GTGGGGGAGT 120

TCCGGGCGGT GACGGAGCTG GGGCGGCCTG ATGAGGAGTA CTGGAACAGC CAGAAGGACC 180

TCCTGGAGGA GAAGCGGGCA GTGCCGGACA GGATGTGCAG ACACAACTAC GAGCTGGTCG 240

GGCCCATGAC CCTGCAG 257

(2) INFORMATION FOR SEQ ID NO:45:
(i) SEQUENCE CHARACTERISTICS:
(A) LENGTH: 85 amino acids
(B) TYPE: amino acid

(C) STRANDEDNESS: single
(D) TOPOLOGY: linear
(ii) MOLECULE TYPE: peptide
(xi) SEQUENCE DESCRIPTION: SEQ ID NO:45:

```
Asn Tyr Leu Phe Gln Gly Arg Gln Glu Cys Tyr Ala Phe Asn Gly Thr
1               5                   10                  15

Gln Arg Phe Leu Glu Arg Tyr Ile Tyr Asn Arg Glu Glu Phe Ala Arg
            20                  25                  30

Phe Asp Ser Asp Val Gly Glu Phe Arg Ala Val Thr Glu Leu Gly Arg
        35                  40                  45

Pro Asp Glu Glu Tyr Trp Asn Ser Gln Lys Asp Leu Leu Glu Glu Lys
    50                  55                  60

Arg Ala Val Pro Asp Arg Met Cys Arg His Asn Tyr Glu Leu Val Gly
65                  70                  75                  80

Pro Met Thr Leu Gln
                85
```

(2) INFORMATION FOR SEQ ID NO:46:
(i) SEQUENCE CHARACTERISTICS:
(A) LENGTH: 257 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:46:

```
AGAATTACCT TTTCCAGGGA CGGCAGGAAT GCTACGCGTT TAATGGGACA CAGCGCTTCC    60

TGGAGAGATA CATCTACAAC CGGGAGGAGT TCGCGCGCTT CGACAGCGAC GTGGGGGAGT   120

TCCGGGCGGT GACGGAGCTG GGGCGGCCTG CTGCGGAGTA CTGGAACAGC CAGAAGGACC   180

TCCTGGAGGA GAAGCGGGCA TTGCCGGACA GGATGTGCAG ACACAACTAC GAGCTGGACG   240

AGGCCGTGAC CCTGCAG                                                  257
```

(2) INFORMATION FOR SEQ ID NO:47:
(i) SEQUENCE CHARACTERISTICS:
(A) LENGTH: 85 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:47:

```
Asn Tyr Leu Phe Gln Gly Arg Gln Glu Cys Tyr Ala Phe Asn Gly Thr
1               5                   10                  15

Gln Arg Phe Leu Glu Arg Tyr Ile Tyr Asn Arg Glu Glu Phe Ala Arg
            20                  25                  30
```

```
Phe Asp Ser Asp Val Gly Glu Phe Arg Ala Val Thr Glu Leu Gly Arg
        35                  40                      45

Pro Ala Ala Glu Tyr Trp Asn Ser Gln Lys Asp Leu Leu Glu Glu Lys
    50                  55                  60

Arg Ala Leu Pro Asp Arg Met Cys Arg His Asn Tyr Glu Leu Asp Glu
65                  70                  75                  80

Ala Val Thr Leu Gln
                85
```

```
(2) INFORMATION FOR SEQ ID NO:48:
     (i) SEQUENCE CHARACTERISTICS:
          (A) LENGTH: 257 base pairs
          (B) TYPE: nucleic acid
          (C) STRANDEDNESS: single
          (D) TOPOLOGY: linear
    (ii) MOLECULE TYPE: DNA (genomic)
    (xi) SEQUENCE DESCRIPTION: SEQ ID NO:48:

AGAATTACGT GTACCAGTTA CGGCAGGAAT GCTACGCGTT TAATGGGACA CAGCGCTTCC      60

TGGAGAGATA CATCTACAAC CGGGAGGAGT ACGCGCGCTT CGACAGCGAC GTGGGGGAGT     120

TCCGGGCGGT GACGGAGCTG GGGCGGCCTG CTGCGGAGTA CTGGAACAGC CAGAAGGACA     180

TCCTGGAGGA GAAGCGGGCA GTGCCGGACA GGATGTGCAG ACACAACTAC GAGCTGGACG     240

AGGCCGTGAC CCTGCAG                                                    257
```

```
(2) INFORMATION FOR SEQ ID NO:49:
     (i) SEQUENCE CHARACTERISTICS:
          (A) LENGTH: 85 amino acids
          (B) TYPE: amino acid
          (C) STRANDEDNESS: single
          (D) TOPOLOGY: linear
    (ii) MOLECULE TYPE: peptide
    (xi) SEQUENCE DESCRIPTION: SEQ ID NO:49:

Asn Tyr Val Tyr Gln Leu Arg Gln Glu Cys Tyr Ala Phe Asn Gly Thr
1               5                   10                  15

Gln Arg Phe Leu Glu Arg Tyr Ile Tyr Asn Arg Glu Glu Tyr Ala Arg
            20                  25                  30

Phe Asp Ser Asp Val Gly Glu Phe Arg Ala Val Thr Glu Leu Gly Arg
        35                  40                      45

Pro Ala Ala Glu Tyr Trp Asn Ser Gln Lys Asp Ile Leu Glu Glu Lys
    50                  55                  60

Arg Ala Val Pro Asp Arg Met Cys Arg His Asn Tyr Glu Leu Asp Glu
65                  70                  75                  80
```

Ala Val Thr Leu Gln
85

(2) INFORMATION FOR SEQ ID NO:50:
(i) SEQUENCE CHARACTERISTICS:
(A) LENGTH: 257 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:50:

AGAATTACCT TTTCCAGGGA CGGCAGGAAT GCTACGCGTT TAATGGGACA CAGCGCTTCC 60

TGGAGAGATA CATCTACAAC CGGGAGGAGT TCGTGCGCTT CGACAGCGAC GTGGGGGAGT 120

TCCGGGCGGT GACGGAGCTG GGGCGGCCTG ATGAGGTGTA CTGGAACAGC CAGAAGGACA 180

TCCTGGAGGA GGAGCGGGCA GTGCCGGACA GGATGTGCAG ACACAACTAC GAGCTGGGCG 240

GGCCCATGAC CCTGCAG 257

(2) INFORMATION FOR SEQ ID NO:51:
(i) SEQUENCE CHARACTERISTICS:
(A) LENGTH: 85 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:51:

Asn Tyr Leu Phe Gln Gly Arg Gln Glu Cys Tyr Ala Phe Asn Gly Thr
1 5 10 15

Gln Arg Phe Leu Glu Arg Tyr Ile Tyr Asn Arg Glu Glu Phe Val Arg
20 25 30

Phe Asp Ser Asp Val Gly Glu Phe Arg Ala Val Thr Glu Leu Gly Arg
35 40 45

Pro Asp Glu Val Tyr Trp Asn Ser Gln Lys Asp Ile Leu Glu Glu Glu
50 55 60

Arg Ala Val Pro Asp Arg Met Cys Arg His Asn Tyr Glu Leu Gly Gly
65 70 75 80

Pro Met Thr Leu Gln
85

(2) INFORMATION FOR SEQ ID NO:52:
(i) SEQUENCE CHARACTERISTICS:
(A) LENGTH: 257 base pairs
(B) TYPE: nucleic acid
(C) STRANDEDNESS: single
(D) TOPOLOGY: linear
(ii) MOLECULE TYPE: DNA (genomic)

(xi) SEQUENCE DESCRIPTION: SEQ ID NO:52:

```
AGAATTACCT TTTCCAGGGA CGGCAGGAAT GCTACGCGTT TAATGGGACA CAGCGCTTCC     60
TGGAGAGATA CATCTACAAC CGGGAGGAGT TCGCGCGCTT CGACAGCGAC GTGGGGGAGT    120
TCCGGGCGGT GACGGAGCTG GGGCGGCCTG CTGCGGAGTA CTGGAACAGC CAGAAGGACA    180
TCCTGGAGGA GGAGCGGGCA GTGCCGGACA GGATGTGCAG ACACAACTAC GAGCTGGGCG    240
GGCCCATGAC CCTGCAG                                                   257
```

(2) INFORMATION FOR SEQ ID NO:53:
(i) SEQUENCE CHARACTERISTICS:
(A) LENGTH: 85 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:53:

```
Asn Tyr Leu Phe Gln Gly Arg Gln Glu Cys Tyr Ala Phe Asn Gly Thr
1               5                   10                  15

Gln Arg Phe Leu Glu Arg Tyr Ile Tyr Asn Arg Glu Glu Phe Ala Arg
            20                  25                  30

Phe Asp Ser Asp Val Gly Glu Phe Arg Ala Val Thr Glu Leu Gly Arg
        35                  40                  45

Pro Ala Ala Glu Tyr Trp Asn Ser Gln Lys Asp Ile Leu Glu Glu Glu
    50                  55                  60

Arg Ala Val Pro Asp Arg Met Cys Arg His Asn Tyr Glu Leu Gly Gly
65                  70                  75                  80

Pro Met Thr Leu Gln
                85
```

(2) INFORMATION FOR SEQ ID NO:54:
(i) SEQUENCE CHARACTERISTICS:
(A) LENGTH: 257 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:54:

```
AGAATTACCT TTTCCAGGGA CGGCAGGAAT GCTACGCGTT TAATGGGACA CAGCGCTTCC     60
TGGAGAGATA CATCTACAAC CGGGAGGAGC TCGTGCGCTT CGACAGCGAC GTGGGGGAGT    120
TCCGGGCGGT GACGGAGCTG GGGCGGCCTG CTGCGGAGTA CTGGAACAGC CAGAAGGACC    180
TCCTGGAGGA GAAGCGGGCA TTGCCGGACA GGATGTGCAG ACACAACTAC GAGCTGGTCG    240
```

GGCCCATGAC CCTGCAG 257

(2) INFORMATION FOR SEQ ID NO:55:
(i) SEQUENCE CHARACTERISTICS:
(A) LENGTH: 85 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:55:

```
Asn Tyr Leu Phe Gln Gly Arg Gln Glu Cys Tyr Ala Phe Asn Gly Thr
1               5                   10                  15

Gln Arg Phe Leu Glu Arg Tyr Ile Tyr Asn Arg Glu Glu Leu Val Arg
            20                  25                  30

Phe Asp Ser Asp Val Gly Glu Phe Arg Ala Val Thr Glu Leu Gly Arg
        35                  40                  45

Pro Ala Ala Glu Tyr Trp Asn Ser Gln Lys Asp Leu Leu Glu Glu Lys
    50                  55                  60

Arg Ala Leu Pro Asp Arg Met Cys Arg His Asn Tyr Glu Leu Val Gly
65                  70                  75                  80

Pro Met Thr Leu Gln
                85
```

(2) INFORMATION FOR SEQ ID NO:56:
(i) SEQUENCE CHARACTERISTICS:
(A) LENGTH: 257 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:56:

```
AGAATTACGT GTACCAGTTA CGGCAGGAAT GCTACGCGTT TAATGGGACA CAGCGCTTCC     60

TGGAGAGATA CATCTACAAC CGGGAGGAGT TCGTGCGCTT CGACAGCGAC GTGGGGGAGT    120

TCCGGGCGGT GACGGAGCTG GGGCGGCCTG ATGAGGACTA CTGGAACAGC CAGAAGGACC    180

TCCTGGAGGA GGAGCGGGCA GTGCCGGACA GGGTATGCAG ACACAACTAC GAGCTGGACG    240

AGGCCGTGAC CCTGCAG                                                  257
```

(2) INFORMATION FOR SEQ ID NO:57:
(i) SEQUENCE CHARACTERISTICS:
(A) LENGTH: 85 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:57:

```
Asn Tyr Val Tyr Gln Leu Arg Gln Glu Cys Tyr Ala Phe Asn Gly Thr
1               5                   10                  15

Gln Arg Phe Leu Glu Arg Tyr Ile Tyr Asn Arg Glu Glu Phe Val Arg
            20                  25                  30

Phe Asp Ser Asp Val Gly Glu Phe Arg Ala Val Thr Glu Leu Gly Arg
        35                  40                  45

Pro Asp Glu Asp Tyr Trp Asn Ser Gln Lys Asp Leu Leu Glu Glu Glu
    50                  55                  60

Arg Ala Val Pro Asp Arg Val Cys Arg His Asn Tyr Glu Leu Asp Glu
65                  70                  75                  80

Ala Val Thr Leu Gln
                85
```

(2) INFORMATION FOR SEQ ID NO:58:
(i) SEQUENCE CHARACTERISTICS:
(A) LENGTH: 257 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:58:

```
AGAATTACGT GCACCAGTTA CGGCAGGAAT GCTACGCGTT TAATGGGACA CAGCGCTTCC    60

TGGAGAGATA CATCTACAAC CGGGAGGAGT TCGTGCGCTT CGACAGCGAC GTGGGGGAGT   120

TCCGGGCGGT GACGGAGCTG GGGCGGCCTG AGGCGGAGTA CTGGAACAGC CAGAAGGACA   180

TCCTGGAGGA GGAGCGGGCA GTGCCGGACA GGATGTGCAG ACACAACTAC GAGCTGGACG   240

AGGCCGTGAC CCTGCAG                                                  257
```

(2) INFORMATION FOR SEQ ID NO:59:
(i) SEQUENCE CHARACTERISTICS:
(A) LENGTH: 85 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:59:

```
Asn Tyr Val His Gln Leu Arg Gln Glu Cys Tyr Ala Phe Asn Gly Thr
1               5                   10                  15

Gln Arg Phe Leu Glu Arg Tyr Ile Tyr Asn Arg Glu Glu Phe Val Arg
            20                  25                  30

Phe Asp Ser Asp Val Gly Glu Phe Arg Ala Val Thr Glu Leu Gly Arg
        35                  40                  45
```

```
Pro Glu Ala Glu Tyr Trp Asn Ser Gln Lys Asp Ile Leu Glu Glu Glu
    50                  55                  60

Arg Ala Val Pro Asp Arg Met Cys Arg His Asn Tyr Glu Leu Asp Glu
65                  70                  75                  80

Ala Val Thr Leu Gln
                85
```

(2) INFORMATION FOR SEQ ID NO:60:
(i) SEQUENCE CHARACTERISTICS:
(A) LENGTH: 257 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:60:

```
AGAATTACGT GCACCAGTTA CGGCAGGAAT GCTACGCGTT TAATGGGACA CAGCGCTTCC    60

TGGAGAGATA CATCTACAAC CGGGAGGAGT TCGTGCGCTT CGACAGCGAC GTGGGGGAGT   120

TCCGGGCGGT GACGGAGCTG GGGCGGCCTG ATGAGGACTA CTGGAACAGC CAGAAGGACA   180

TCCTGGAGGA GAAGCGGGCA GTGCCGGACA GGGTATGCAG ACACAACTAC GAGCTGGACG   240

AGGCCGTGAC CCTGCAG                                                  257
```

(2) INFORMATION FOR SEQ ID NO:61:
(i) SEQUENCE CHARACTERISTICS:
(A) LENGTH: 85 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:61:

```
Asn Tyr Val His Gln Leu Arg Gln Glu Cys Tyr Ala Phe Asn Gly Thr
1               5                   10                  15

Gln Arg Phe Leu Glu Arg Tyr Ile Tyr Asn Arg Glu Glu Phe Val Arg
            20                  25                  30

Phe Asp Ser Asp Val Gly Glu Phe Arg Ala Val Thr Glu Leu Gly Arg
        35                  40                  45

Pro Asp Glu Asp Tyr Trp Asn Ser Gln Lys Asp Ile Leu Glu Glu Lys
    50                  55                  60

Arg Ala Val Pro Asp Arg Val Cys Arg His Asn Tyr Glu Leu Asp Glu
65                  70                  75                  80

Ala Val Thr Leu Gln
                85
```

(2) INFORMATION FOR SEQ ID NO:62:
(i) SEQUENCE CHARACTERISTICS:
(A) LENGTH: 257 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:62:

AGAATTACGT GTCCCAGTTA CGGCAGGAAT GCTACGCGTT TAATGGGACA CAGCGCTTCC 60

TGGAGAGATA CATCTACAAC CGGGAGGAGC TCGTGCGCTT CGACAGCGAC GTGGGGGAGT 120

TCCGGGCGGT GACGGAGCTG GGGCGGCCTG AGGCGGAGTA CTGGAACAGC CAGAAGGACA 180

TCCTGGAGGA GGAGCGGGCA GTGCCGGACA GGATGTGCAG ACACAACTAC GAGCTGGACG 240

AGGCCGTGAC CCTGCAG 257

(2) INFORMATION FOR SEQ ID NO:63:
(i) SEQUENCE CHARACTERISTICS:
(A) LENGTH: 85 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:63:

Asn Tyr Val His Gln Leu Arg Gln Glu Cys Tyr Ala Phe Asn Gly Thr
1 5 10 15

Gln Arg Phe Leu Glu Arg Tyr Ile Tyr Asn Arg Glu Glu Leu Val Arg
20 25 30

Phe Asp Ser Asp Val Gly Glu Phe Arg Ala Val Thr Glu Leu Gly Arg
35 40 45

Pro Glu Ala Glu Tyr Trp Asn Ser Gln Lys Asp Ile Leu Glu Glu Glu
50 55 60

Arg Ala Val Pro Asp Arg Met Cys Arg His Asn Tyr Glu Leu Asp Glu
65 70 75 80

Ala Val Thr Leu Gln
85

(2) INFORMATION FOR SEQ ID NO: 64:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 64:
TACCTTTTCC AGGGACGG 18

(2) INFORMATION FOR SEQ ID NO: 65:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 65:
TACGTGTACC AGTTACGG 18

(2) INFORMATION FOR SEQ ID NO: 66:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 66:
TACGTGTACC AGGGACGG 18

(2) INFORMATION FOR SEQ ID NO: 67:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 67:
TACGTGCACC AGTTACGG 18

(2) INFORMATION FOR SEQ ID NO: 68:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 68:
CGGGAGGAGT TCGCGCGC 18

(2) INFORMATION FOR SEQ ID NO: 69:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 69:
CGGGAGGAGT TCGTGCGC 18

(2) INFORMATION FOR SEQ ID NO: 70:
(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: genomic DNA
(xi) SEQUENCE DESCRIPTION: SEQ ID NO: 70:
CGGGAGGAGC TCGTGCGC 18

(2) INFORMATION FOR SEQ ID NO: 71:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 71:
CGGCAGGAGT ACGCGCGC 18

(2) INFORMATION FOR SEQ ID NO: 72:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 72:
CGGGAGGAGT ACGCGCGC 18

(2) INFORMATION FOR SEQ ID NO: 73:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 73:
CGGGAGGAAT TCGTGCGC 18

(2) INFORMATION FOR SEQ ID NO: 74:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 74:
CCTGCTGCGG AGTACTGG 18

(2) INFORMATION FOR SEQ ID NO: 75:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 75:
CCTGATGAGG AGTACTGG 18

(2) INFORMATION FOR SEQ ID NO: 76:
(i) SEQUENCE CHARACTERISTICS:

(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 76:
CCTGAGGCGG AGTACTGG 18

(2) INFORMATION FOR SEQ ID NO: 77:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 77:
CCTGATGAGG ACTACTGG 18

(2) INFORMATION FOR SEQ ID NO: 78:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 78:
GACATCCTGG AGGAGAAG 18

(2) INFORMATION FOR SEQ ID NO: 79:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 79:
GACATCCTGG AGGAGGAG 18

(2) INFORMATION FOR SEQ ID NO: 80:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 80:
GACCTCCTGG AGGAGAAG 18

(2) INFORMATION FOR SEQ ID NO: 81:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA

(xi) SEQUENCE DESCRIPTION: SEQ ID NO: 81:
GACCTCCTGG AGGAGGAG 18

(2) INFORMATION FOR SEQ ID NO: 82:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 82:
GACCTCCTGG AGGAGAGG 18

(2) INFORMATION FOR SEQ ID NO: 83:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 83:
GACAGGATGT GCAGACAC 18

(2) INFORMATION FOR SEQ ID NO: 84:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 84:
GACAGGGTAT GCAGACAC 18

(2) INFORMATION FOR SEQ ID NO: 85:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 85:
CTGGGCGGGC CCATGACC 18

(2) INFORMATION FOR SEQ ID NO: 86:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 86:
CTGGACGAGG CCGTGACC 18

(2) INFORMATION FOR SEQ ID NO: 87:
(i) SEQUENCE CHARACTERISTICS:
(A) LENGTH: 18 base pairs

```
        (B) TYPE: nucleic acid
        (C) STRANDEDNESS: single
        (D) TOPOLOGY: linear
    (ii) MOLECULE TYPE: genomic DNA
    (xi) SEQUENCE DESCRIPTION: SEQ ID NO: 87:
CTGGTCGGGC CCATGACC                                         18

(2) INFORMATION FOR SEQ ID NO: 88:
    (i) SEQUENCE CHARACTERISTICS:
        (A) LENGTH: 19 base pairs
        (B) TYPE: nucleic acid
        (C) STRANDEDNESS: single
        (D) TOPOLOGY: linear
    (ii) MOLECULE TYPE: genomic DNA
    (xi) SEQUENCE DESCRIPTION: SEQ ID NO: 88:
TGTCTGCACA TCCTGTCCG                                        19

(2) INFORMATION FOR SEQ ID NO: 89:
    (i) SEQUENCE CHARACTERISTICS:
        (A) LENGTH: 19 base pairs
        (B) TYPE: nucleic acid
        (C) STRANDEDNESS: single
        (D) TOPOLOGY: linear
    (ii) MOLECULE TYPE: genomic DNA
    (xi) SEQUENCE DESCRIPTION: SEQ ID NO: 89:
TGTCTGCATA CCCTGTCCG                                        19

(2) INFORMATION FOR SEQ ID NO: 90:
    (i) SEQUENCE CHARACTERISTICS:
        (A) LENGTH: 19 base pairs
        (B) TYPE: nucleic acid
        (C) STRANDEDNESS: single
        (D) TOPOLOGY: linear
    (ii) MOLECULE TYPE: genomic DNA
    (xi) SEQUENCE DESCRIPTION: SEQ ID NO: 90:
CGGACAGGAT ATGCAGACA                                        19

(2) INFORMATION FOR SEQ ID NO: 91:
    (i) SEQUENCE CHARACTERISTICS:
        (A) LENGTH: 28 base pairs
        (B) TYPE: nucleic acid
        (C) STRANDEDNESS: single
        (D) TOPOLOGY: linear
    (ii) MOLECULE TYPE: genomic DNA
    (xi) SEQUENCE DESCRIPTION: SEQ ID NO: 91:
GGGATCCGAG AGTGGCGCCT CCGCTCAT                              28

(2) INFORMATION FOR SEQ ID NO: 92:
    (i) SEQUENCE CHARACTERISTICS:
        (A) LENGTH: 17 base pairs
        (B) TYPE: nucleic acid
        (C) STRANDEDNESS: single
        (D) TOPOLOGY: linear
    (ii) MOLECULE TYPE: genomic DNA
    (xi) SEQUENCE DESCRIPTION: SEQ ID NO: 92:
CCTGATGAGG TGTACTG                                          17
```

(2) INFORMATION FOR SEQ ID NO: 93:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 93:
AGATGAGATG TTCTATG 17

(2) INFORMATION FOR SEQ ID NO: 94:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 94:
GTTTGGCCAA GCCTTTT 17

(2) INFORMATION FOR SEQ ID NO: 95:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 95:
AGATGAGCAG TTCTATG 17

(2) INFORMATION FOR SEQ ID NO: 96:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 96:
GTTTGGCCGA GCCTTTT 17

(2) INFORMATION FOR SEQ ID NO: 97:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 97:
CAGGGATCCG CAGAGAATTA C 21

(2) INFORMATION FOR SEQ ID NO: 98:
(i) SEQUENCE CHARACTERISTICS:
(A) LENGTH: 24 base pairs
(B) TYPE: nucleic acid
(C) STRANDEDNESS: single

(D) TOPOLOGY: linear
(ii) MOLECULE TYPE: genomic DNA
(xi) SEQUENCE DESCRIPTION: SEQ ID NO: 98:
GTCCTGCAGT CACTCACCTC GGCG 24

(2) INFORMATION FOR SEQ ID NO: 99:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 99:
GAATTACCTT TTCCAGGGA 19

(2) INFORMATION FOR SEQ ID NO: 100:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 100:
ATTACGTGTA CCAGTTACG 19

(2) INFORMATION FOR SEQ ID NO: 101:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 101:
CGTCCCTGGT ACACGTAAT 19

(2) INFORMATION FOR SEQ ID NO: 102:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 102:
CCTGCTGCGG AGTACTG 17

(2) INFORMATION FOR SEQ ID NO: 103:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 103:
CAGTACTCCT CATCAGG 17

(2) INFORMATION FOR SEQ ID NO: 104:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 104:
CAGTACTCCG CCTCAGG 17

(2) INFORMATION FOR SEQ ID NO: 105:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 105:
CCTGATGAGG ACTACTG 17

(2) INFORMATION FOR SEQ ID NO: 106:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 106:
GACATCCTGG AGGAGAAGC 19

(2) INFORMATION FOR SEQ ID NO: 107:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 107:
GCTCCTCCTC CAGGATGTC 19

(2) INFORMATION FOR SEQ ID NO: 108:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 108:
GACCTCCTGG AGGAGAAGC 19

(2) INFORMATION FOR SEQ ID NO: 109:
(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: genomic DNA
(xi) SEQUENCE DESCRIPTION: SEQ ID NO: 109:
GCTCCTCCTC CAGGAGGTC 19

(2) INFORMATION FOR SEQ ID NO: 110:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 110:
ATTACGTGCA CCAGTTACG 19

(2) INFORMATION FOR SEQ ID NO: 111:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 111:
CGTAACTGGT ACACGTAAT 19

(2) INFORMATION FOR SEQ ID NO: 112:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 112:
CTGCAGGGTC ATGGGCCCCC G 21

(2) INFORMATION FOR SEQ ID NO: 113:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 113:
CTGCAGGGTC ACGGCCTCGT C 21

(2) INFORMATION FOR SEQ ID NO: 114:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 114:
ATTACGTGTA CCAGTTA 17

(2) INFORMATION FOR SEQ ID NO: 115:
(i) SEQUENCE CHARACTERISTICS:

(A) LENGTH: 17 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 115:
CCTGAGGCGG AGTACTG 17

(2) INFORMATION FOR SEQ ID NO: 116:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 116:
GACCTCCTGG AGGAGGAG 18

(2) INFORMATION FOR SEQ ID NO: 117:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 117:
GACCTCCTGG AGGAGAGG 18

(2) INFORMATION FOR SEQ ID NO: 118:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 118:
CTGGTCGGGC CCATGACC 18

(2) INFORMATION FOR SEQ ID NO: 119:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 119:
GAATTACCTT TTCCAGGGAC 20

(2) INFORMATION FOR SEQ ID NO: 120:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: genomic DNA

(xi) SEQUENCE DESCRIPTION: SEQ ID NO: 120:
TTACGTGTAC CTGGGAC 17

(2) INFORMATION FOR SEQ ID NO: 121:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 121:
ACATCCTGGA GGAGAAGC 18

(2) INFORMATION FOR SEQ ID NO: 122:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 122:
ACATCCTGGA GGAGGAGC 18

(2) INFORMATION FOR SEQ ID NO: 123:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 123:
ACCTCCTGGA GGAGAAGC 18

(2) INFORMATION FOR SEQ ID NO: 124:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 124:
CCTGATGAGG AGTACTG 17

(2) INFORMATION FOR SEQ ID NO: 125:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 125:
CTGGGCGGGC CCATG 15

(2) INFORMATION FOR SEQ ID NO: 126:
(i) SEQUENCE CHARACTERISTICS:
(A) LENGTH: 15 base pairs

(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 126:
CTGGACGAGG CCGTG 15

(2) INFORMATION FOR SEQ ID NO: 127:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 127:
GACCTCCTGG AGGAGGAGC 19

(2) INFORMATION FOR SEQ ID NO: 128:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 128:
GACCTCCTGG AGGAGAGGC 19

(2) INFORMATION FOR SEQ ID NO: 129:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 129:
AGCTGGGCGG GCCCATGAC 19

(2) INFORMATION FOR SEQ ID NO: 130:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 130:
AGCTGGACGA GGCCGTGAC 24

(2) INFORMATION FOR SEQ ID NO: 131:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 131:
CCAGTACTCC TCATCAGGC 19

(2) INFORMATION FOR SEQ ID NO: 132:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 132:
ATTACGTGCA CCAGTTAC 18

(2) INFORMATION FOR SEQ ID NO: 133:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 133:
ATTACGTGCA CCAGTTA 17

(2) INFORMATION FOR SEQ ID NO: 134:
(i) SEQUENCE CHARACTERISTICS:
(A) LENGTH: 16 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 134:
CAGTACTCCT CATCAG 16

(2) INFORMATION FOR SEQ ID NO: 135:
(i) SEQUENCE CHARACTERISTICS:
(A) LENGTH: 16 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 135:
CTGATGAGGA CTACTG 16

(2) INFORMATION FOR SEQ ID NO: 136:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 136:
CGCTTCGACA GCGACGT 17

(2) INFORMATION FOR SEQ ID NO: 137:
(i) SEQUENCE CHARACTERISTICS:
(A) LENGTH: 22 base pairs
(B) TYPE: nucleic acid
(C) STRANDEDNESS: single

(D) TOPOLOGY: linear
(ii) MOLECULE TYPE: genomic DNA
(xi) SEQUENCE DESCRIPTION: SEQ ID NO: 137:
CCGTCCCTGG AAAAGGTAAT TC 22

(2) INFORMATION FOR SEQ ID NO: 138:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 138:
GACCTCCTGN GAGGAGAGGC 20

(2) INFORMATION FOR SEQ ID NO: 139:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 139:
GACCTCCTGG AGNGAGGAGC 20

(2) INFORMATION FOR SEQ ID NO: 140:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 140:
CGCGGATCCT GTGTCAACTT ATGCCGC 27

(2) INFORMATION FOR SEQ ID NO: 141:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 141:
CTGGCTGCAG TGTGGTTGGA ACGC 24

(2) INFORMATION FOR SEQ ID NO: 142:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 142:
ACACAGGAAA CAGCTATGAC CATG 24

(2) INFORMATION FOR SEQ ID NO: 143:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 143:
CCAGGGTTTT CCCAGTCACG AC 22

(2) INFORMATION FOR SEQ ID NO: 144:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 144:
GCTGCAGGAG AGTGGCGCCT CCGCTCAT 28

(2) INFORMATION FOR SEQ ID NO: 145:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 145:
CGGATCCGGC CCAAAGCCCT CACTC 25

(2) INFORMATION FOR SEQ ID NO: 146:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 146:
GCGGCATAAG TTGACACATG GTCCGCT 27

(2) INFORMATION FOR SEQ ID NO: 147:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 147:
GCGTTCCAAC CACACTCAGG CCAC 24

(2) INFORMATION FOR SEQ ID NO: 148:
(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: genomic DNA
(xi) SEQUENCE DESCRIPTION: SEQ ID NO: 148:
GTAATTCTCT GCGGGGAGGG G 21

(2) INFORMATION FOR SEQ ID NO: 149:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 149:
CGCCGAGGTG AGTGAGGGCT TTGG 24

(2) INFORMATION FOR SEQ ID NO: 150:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 150:
GACAGGATAT GCAGACAC 18

(2) INFORMATION FOR SEQ ID NO: 151:
(i) SEQUENCE CHARACTERISTICS:
(A) LENGTH: 16 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 151:
AGGAGTTCGC GCGCTT 16

(2) INFORMATION FOR SEQ ID NO: 152:
(i) SEQUENCE CHARACTERISTICS:
(A) LENGTH: 16 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 152:
AGGAGTTCGT GCGCTT 16

(2) INFORMATION FOR SEQ ID NO: 153:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 153:
AGGAGCTCGT GCGCTTC 17

(2) INFORMATION FOR SEQ ID NO: 154:
(i) SEQUENCE CHARACTERISTICS:

(A) LENGTH: 17 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 154:
CCGGCAGGAG TACGCGC 17

(2) INFORMATION FOR SEQ ID NO: 155:
(i) SEQUENCE CHARACTERISTICS:
(A) LENGTH: 16 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 155:
GAGGAGTACG CGCGCT 16

(2) INFORMATION FOR SEQ ID NO: 156:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 156:
CGAGCTGGGC GGGCCCA 17

(2) INFORMATION FOR SEQ ID NO: 157:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: genomic DNA
(xi) SEQUENCE DESCRIPTION: SEQ ID NO: 157:
CGAGCTGGTC GGGCCCA 17

## Claims

1. A process for determining an individual's HLA DP genotype from a nucleic acid containing sample originating from the individual whose HLA-DP genotype is to be determined, which method comprises:
(a) amplifying a target region of the nucleic acids in the sample under conditions suitable for carrying out a polymerase chain reaction, whereby the target region contains a polymorphic region (variable segment) of an HLA DP gene using a primer selected from the group consisting of primers

AB111 SEQ ID NO: 91 5'GGGATCCGAGAGTGGCGCCTCCGCTCAT,
RS348 SEQ ID NO: 142 5'ACACAGGAAACAGCTATGACCATG, and
RS349 SEQ ID NO: 143 5'CCAGGGTTTTCCCAGTCACGAC;

(b) mixing the amplified nucleic acids with a panel of sequence specific oligonucleotide (SSO) probes, wherein each probe is complementary to a variant sequence of a variable segment of an HLA DP gene, under conditions wherein SSO probes bind to said amplified nucleic acids to form stable hybrid duplexes only if they are exactly complementary; and
(c) detecting hybrids formed between the amplified nucleic acids and the SSO probes.

2. The process of Claim 1, wherein the probes of the panel are selected from the group consisting of:

SEQ ID NO: 92 (5'CCTGATGAGGTGTACTG);
SEQ ID NO: 99 (5'GAATTACCTTTTCCAGGGA);
SEQ ID NO: 100 (5'ATTACGTGTACCAGTTACG);
SEQ ID NO: 101 (5'CGTCCCTGGTACACGTAAT);
SEQ ID NO: 102 (5'CCTGCTGCGGAGTACTG);
SEQ ID NO: 103 (5'CAGTACTCCTCATCAGG);
SEQ ID NO: 104 (5'CAGTACTCCGCCTCAGG);
SEQ ID NO: 105 (5'CCTGATGAGGACTACTG);
SEQ ID NO: 106 (5'GACATCCTGGAGGAGAAGC);
SEQ ID NO: 107 (5'GCTCCTCCTCCAGGATGTC);
SEQ ID NO: 108 (5'GACCTCCTGGAGGAGAAGC);
SEQ ID NO: 109 (5'GCTCCTCCTCCAGGAGGTC);
SEQ ID NO: 110 (5'ATTACGTGCACCAGTTACG);
SEQ ID NO: 111 (5'CGTAACTGGTACACGTAAT);

SEQ ID NO: 112 (5'CTGCAGGGTCATGGGCCCCCG);
SEQ ID NO: 113 (5'CTGCAGGGTCACGGCCTCGTC);
SEQ ID NO: 114 (5'ATTACGTGTACCAGTTA);
SEQ ID NO: 115 (5'CCTGAGGCGGAGTACTG);
SEQ ID NO: 116 (5'GACCTCCTGGAGGAGGAG);
SEQ ID NO: 117 (5'GACCTCCTGGAGGAGAGG);
SEQ ID NO: 118 (5'CTGGTCGGGCCCATGACC);
SEQ ID NO: 119 (5'GAATTACCTTTTCCAGGGAC);
SEQ ID NO: 120 (5'TTACGTGTACCTGGGAC);
SEQ ID NO: 121 (5'ACATCCTGGAGGAGAAGC);
SEQ ID NO: 122 (5'ACATCCTGGAGGAGGAGC);
SEQ ID NO: 123 (5'ACCTCCTGGAGGAGAAGC);
SEQ ID NO: 124 (5'CCTGATGAGGAGTACTG);
SEQ ID NO: 125 (5'CTGGGCGGGCCCATG);
SEQ ID NO: 126 (5'CTGGACGAGGCCGTG);
SEQ ID NO: 127 (5'GACCTCCTGGAGGAGGAGC);
SEQ ID NO: 128 (5'GACCTCCTGGAGGAGAGGC);
SEQ ID NO: 129 (5'AGCTGGGCGGGCCCATGAC);
SEQ ID NO: 130 (5'AGCTGGACGAGGCCGTGAC);
SEQ ID NO: 132 (5'ATTACGTGCACCAGTTAC);
SEQ ID NO: 133 (5'ATTACGTGCACCAGTTA);
SEQ ID NO: 134 (5'CAGTACTCCTCATCAG);
SEQ ID NO: 135 (5'CTGATGAGGACTACTG);
SEQ ID NO: 137 (5'CCGTCCCTGGAAAAGGTAATTC);
SEQ ID NO: 138 (5'GACCTCCTGNGAGGAGAGGC);
SEQ ID NO: 139 (5'GACCTCCTGGAGNGAGGAGC);
SEQ ID NO: 151 (X-AGGAGTTCGCGCGCTT);
SEQ ID NO: 152 (X-AGGAGTTCGTGCGCTT);
SEQ ID NO: 153 (X-AGGAGCTCGTGCGCTTC);
SEQ ID NO: 154 (X-CCGGCAGGAGTACGCGC);
SEQ ID NO: 155 (X-GAGGAGTACGCGCGCT);
SEQ ID NO: 156 (X-CGAGCTGGGCGGGCCCA); and
SEQ ID NO: 157 (X-CGAGCTGGTCGGGCCCA).

**3.** The process of Claim 1, wherein the probes of the panel are selected from the group consisting of:

SEQ ID NO: 92 (5'CCTGATGAGGTGTACTG);
SEQ ID NO: 132 (5'ATTACGTGCACCAGTTAC);

SEQ ID NO: 133 (5'ATTACGTGCACCAGTTA);
SEQ ID NO: 134 (5'CAGTACTCCTCATCAG);
SEQ ID NO: 135 (5'CTGATGAGGACTACTG);
SEQ ID NO: 137 (5'CCGTCCCTGGAAAAGGTAATTC);
SEQ ID NO: 138 (5'GACCTCCTGNGAGGAGAGGC);
SEQ ID NO: 139 (5'GACCTCCTGGAGNGAGGAGC);
SEQ ID NO: 151 (X-AGGAGTTCGCGCGCTT);
SEQ ID NO: 152 (X-AGGAGTTCGTGCGCTT);
SEQ ID NO: 153 (X-AGGAGCTCGTGCGCTTC);
SEQ ID NO: 154 (X-CCGGCAGGAGTACGCGC);
SEQ ID NO: 155 (X-GAGGAGTACGCGCGCT);
SEQ ID NO: 156 (X-CGAGCTGGGCGGGCCCA); and
SEQ ID NO: 157 (X-CGAGCTGGTCGGGCCCA).

4. The process of Claim 1, wherein the individual's HLA DP genotype comprises an allele selected from the group consisting of DPB21, DPB22, DPB23, DPB24, DPB25, DPB26, DPB27, DPB28, DPB29, and DPB30.

5. A process for determining an individual's susceptibility to an autoimmune disease comprising determining the individual's HLA DP genotype according to the process of Claim 1 and determining whether the individual's genotype is a genotype which is linked to an autoimmune disease.

6. The process of Claim 5, wherein the autoimmune disease is pauciarticular juvenile rheumatoid arthritis, and wherein the genotype which is linked to an autoimmune disease comprises the DPB2.1 allele.

7. The process of Claim 6, wherein the autoimmune disease is IDDM.

8. The process of Claim 6, wherein the autoimmune disease is CD, and wherein the genotype which is linked to an autoimmune disease comprises an allele selected from the group consisting of DPB13, DPB1, DPB3, and DPB4.2.

9. A process of providing forensic evidence concerning the derivation of a sample which contains genomic nucleic acids comprising determining, according to the process in Claim 1, the HLA DP genotypes of the sample and of a suspected individual, comparing the HLA DP genotypes of the individual and of the sample, and deducing whether the sample could have been derived from the individual.

10. The process of Claim 1, wherein the panel of probes comprises probes with hybridizing regions:

SEQ ID NO: 88 (5'TGTCTGCACATCCTGTCCG);
SEQ ID NO: 89 (5'TGTCTGCATACCCTGTCCG);
SEQ ID NO: 90 (5'CGGACAGGATATGCAGACA);
SEQ ID NO: 99 (5'GAATTACCTTTTCCAGGGA);
SEQ ID NO: 101 (5'CGTCCCTGGTACACGTAAT);
SEQ ID NO: 102 (5'CCTGCTGCGGAGTACTG);
SEQ ID NO: 105 (5'CCTGATGAGGACTACTG);
SEQ ID NO: 106 (5'GACATCCTGGAGGAGAAGC);
SEQ ID NO: 107 (5'GCTCCTCCTCCAGGATGTC);
SEQ ID NO: 108 (5'GACCTCCTGGAGGAGAAGC);
SEQ ID NO: 110 (5'ATTACGTGCACCAGTTACG);
SEQ ID NO: 112 (5'CTGCAGGGTCATGGGCCCCCG);
SEQ ID NO: 114 (5'ATTACGTGTACCAGTTA);
SEQ ID NO: 115 (5'CCTGAGGCGGAGTACTG);
SEQ ID NO: 116 (5'GACCTCCTGGAGGAGGAG);
SEQ ID NO: 117 (5'GACCTCCTGGAGGAGAGG);
SEQ ID NO: 126 (5'CTGGACGAGGCCGTG); and
SEQ ID NO: 131 (5'CCAGTACTCCTCATCAGGC).

**11.** The process of Claim 10, wherein the panel of probes further comprises a probe with hybridizing region SEQ ID NO: 92 (5'CCTGATGAGGTGTACTG).

**12.** The process of Claim 10, wherein the primers used are

UG19 SEQ ID NO: 144 (GCTGCAGGAGAGTGGCGCCTCCGCTCAT) and
UG21 SEQ ID NO: 145 (CGGATCCGGCCCAAAGCCCTCACTC).

**13.** An oligonucleotide probe selected from the group consisting of:

SEQ ID NO: 92 (5'CCTGATGAGGTGTACTG);
SEQ ID NO: 132 (5'ATTACGTGCACCAGTTAC);
SEQ ID NO: 133 (5'ATTACGTGCACCAGTTA);

SEQ ID NO: 134 (5'CAGTACTCCTCATCAG);
SEQ ID NO: 135 (5'CTGATGAGGACTACTG);
SEQ ID NO: 137 (5'CCGTCCCTGGAAAAGGTAATTC);
SEQ ID NO: 138 (5'GACCTCCTGNGAGGAGAGGC);
SEQ ID NO: 139 (5'GACCTCCTGGAGNGAGGAGC);
SEQ ID NO: 151 (X-AGGAGTTCGCGCGCTT);
SEQ ID NO: 152 (X-AGGAGTTCGTGCGCTT);
SEQ ID NO: 153 (X-AGGAGCTCGTGCGCTTC);
SEQ ID NO: 154 (X-CCGGCAGGAGTACGCGC);
SEQ ID NO: 155 (X-GAGGAGTACGCGCGCT);
SEQ ID NO: 156 (X-CGAGCTGGGCGGGCCCA); and
SEQ ID NO: 157 (X-CGAGCTGGTCGGGCCCA).

**14.** A primer selected from the group consisting of primers

| | | |
|---|---|---|
| AB111 | SEQ ID NO: 91 | 5'GGGATCCGAGAGTGGCGCCTCCGCTCAT, |
| RS348 | SEQ ID NO: 142 | 5'ACACAGGAAACAGCTATGACCATG, and |
| RS349 | SEQ ID NO: 143 | 5'CCAGGGTTTTCCCAGTCACGAC. |

**15.** An oligonucleotide probe as claimed in claim 13 as a diagnostic tool.

**16.** A primer as claimed in claim 14 as a diagnostic tool.

**17.** A panel of SSO probes, wherein the probes of the panel are selected from the group consisting of:

SEQ ID NO: 92 (5'CCTGATGAGGTGTACTG);
SEQ ID NO: 132 (5'ATTACGTGCACCAGTTAC);
SEQ ID NO: 133 (5'ATTACGTGCACCAGTTA);
SEQ ID NO: 134 (5'CAGTACTCCTCATCAG);
SEQ ID NO: 135 (5'CTGATGAGGACTACTG);
SEQ ID NO: 137 (5'CCGTCCCTGGAAAAGGTAATTC);
SEQ ID NO: 138 (5'GACCTCCTGNGAGGAGAGGC);
SEQ ID NO: 139 (5'GACCTCCTGGAGNGAGGAGC);
SEQ ID NO: 151 (X-AGGAGTTCGCGCGCTT);
SEQ ID NO: 152 (X-AGGAGTTCGTGCGCTT);
SEQ ID NO: 153 (X-AGGAGCTCGTGCGCTTC);
SEQ ID NO: 154 (X-CCGGCAGGAGTACGCGC);
SEQ ID NO: 155 (X-GAGGAGTACGCGCGCT);

SEQ ID NO: 156 (X-CGAGCTGGGCGGGCCCA); and
SEQ ID NO: 157 (X-CGAGCTGGTCGGGCCCA).

**18.** The use of an oligonucleotide probe as claimed in claim 13, a primer as claimed in claim 14, or a panel of SSO probes as claimed in claim 17 for determining an individual's HLA DP genotype.

**19.** The use of an oligonucleotide probe as claimed in claim 13, a primer as claimed in claim 14, or a panel of SSO probes as claimed in claim 17 for determining an individual's HLA DP genotype, wherein the individual's HLA DP genotype comprises an allele selected from the group consisting of DPB21, DPB22, DPB23, DPB24, DPB25, DPB26, DPB27, DPB28, DPB29, and DPB30.

**20.** A kit useful for determining an individual's HLA DP genotype comprising:
(a) a panel of SSO probes, wherein the probes of the panel are selected from the group consisting of:

SEQ ID NO: 92 (5'CCTGATGAGGTGTACTG);
SEQ ID NO: 132 (5'ATTACGTGCACCAGTTAC);
SEQ ID NO: 133 (5'ATTACGTGCACCAGTTA);
SEQ ID NO: 134 (5'CAGTACTCCTCATCAG);
SEQ ID NO: 135 (5'CTGATGAGGACTACTG);
SEQ ID NO: 137 (5'CCGTCCCTGGAAAAGGTAATTC);
SEQ ID NO: 138 (5'GACCTCCTGNGAGGAGAGGC);
SEQ ID NO: 139 (5'GACCTCCTGGAGNGAGGAGC);
SEQ ID NO: 151 (X-AGGAGTTCGCGCGCTT);
SEQ ID NO: 152 (X-AGGAGTTCGTGCGCTT);
SEQ ID NO: 153 (X-AGGAGCTCGTGCGCTTC);
SEQ ID NO: 154 (X-CCGGCAGGAGTACGCGC);
SEQ ID NO: 155 (X-GAGGAGTACGCGCGCT);
SEQ ID NO: 156 (X-CGAGCTGGGCGGGCCCA); and
SEQ ID NO: 157 (X-CGAGCTGGTCGGGCCCA);

as well as
(b) instructions for determining the genotype by utilizing kit ingredients.

**21.** The kit of Claim 20, wherein the individual's HLA DP genotype comprises an allele selected from the group consisting of DPB21, DPB22, DPB23, DPB24, DPB25, DPB26, DPB27, DPB28, DPB29, and DPB30.

**22.** The kit of Claim 20 further comprising a container containing oligonucleotide primers useful for amplification of a target region of an HLA DP gene, wherein said target region contains said variable segment.